# EUROPEAN PATENT SPECIFICATION

(11) **EP 3 918 088 B1**
(45) Date of publication and mention of the grant of the patent: **13.03.2024**
(21) Application number: 20749055.8
(22) Date of filing: 29.01.2020
(51) Int. Cl.: C12Q 1/68, C40B 50/06

(54) **HIGH COVERAGE STLFR**
STLFR MIT HOHER ABDECKUNG
STLFR À COUVERTURE ÉLEVÉE

(30) Priority: 29.01.2019 US 201962798378 P; 06.05.2019 US 201962843972 P
(43) Date of publication of application: 08.12.2021
(73) Proprietor: MGI Tech Co., Ltd., Shenzhen, Guangdong 518083 (CN); BGI Shenzhen, Shenzhen, Guangdong 518083 (CN)
(72) Inventor: PETERS, Brock A., San Jose, California 95134 (US); WANG, Ou, San Jose, California 95134 (US); DRMANAC, Radoje T., San Jose, California 95134 (US)
(74) Representative: Patent Boutique LLP
(86) International application number: PCT/IB2020/050715
(87) International publication number: WO 2020/157684

(56) References cited:
- WO-A1-2015/085274
- WO-A1-2017/151828
- WO-A1-2018/118971
- WO-A1-2019/217452
- WO-A2-2014/145820
- WO-A2-2014/145820
- WO-A2-2015/051006
- WO-A2-2016/061517
- WO-A2-2016/109604
- CN-A- 106 795 651

## Description

### BACKGROUND

To date the vast majority of individual whole genome sequences lack information regarding the order of single to multi-base variants transmitted as contiguous blocks on homologous chromosomes. Numerous technologies have recently been developed to enable this. Most are based on the process of co-barcoding (Peters et al., Frontiers in Genetics 5, 466 (2014)), that is, the addition of the same barcode to the sub-fragments of single long genomic DNA molecules. After sequencing, the barcode information can be used to determine which reads are derived from the original long DNA molecule. This process was first described by Drmanac (WO2006/138284 A2 (2006)) and implemented as a 384-well plate assay by Peters et al. (Peters et al., Nature 487, 190-195 (2012)) and later implemented in single reaction vessel format in Drmanac (US 2014/0323316) and Wang et al., BioRxiv, June 29, 2018, doi:https//doi.org/10.1101/357863. WO 2014/145820 A2 describes methods and compositions for tagging long fragments of a target nucleic acid for sequencing and analyzing the resulting sequence information. WO 2016/061517 A2 describes methods and compositions for preparing an immobilized library of barcoded DNA fragments of a target nucleic acid, identifying genomic variants, determining the contiguity information, phasing information and methylation status of the target nucleic acid. WO 2018/118971 A1 describes methods and compositions for maintaining DNA contiguity for sequencing.

### SUMMARY OF INVENTION

The invention provides a method for preparing a sequencing library for sequencing a target nucleic acid as specified by claim 1. Further features of the invention are defined in the dependent claims. Methods not encompassed by the wording of the claims but are considered as useful for understanding the invention include a method for preparing a library of barcoded polynucleotides for determining a sequence of a target nucleic acid(s) comprising
(a) providing fragments derived from the target nucleic acid, wherein the fragments are double-stranded or partially double-stranded;
(b) introducing staggered single-stranded breaks into at least some double-stranded fragments, thereby producing a plurality of first complexes, wherein each first complex comprises a plurality of first subfragments, and
(c) associating first capture oligonucleotide sequences with at least some of the first subfragments,
   wherein each first capture oligonucleotide sequence comprises a first barcode, and optionally comprises a promoter sequence or a primer binding sequence, and
   wherein the associating comprises combining the double-stranded fragments in (a) or the complexes in (b) with a plurality of individual first beads, wherein each individual first bead comprises a plurality of first capture oligonucleotides immobilized thereon, wherein each capture oligonucleotides comprises a first capture oligonucleotide sequence, wherein the first capture oligonucleotides immobilized on each individual first bead comprises the same first capture oligonucleotide sequence, wherein a majority of different first beads have different first capture oligonucleotides immobilized thereon, and wherein each different first capture oligonucleotide sequence comprises a different first barcode,
   thereby providing barcoded first subfragments;
(d) amplifying at least a portion of the barcoded first subfragments to produce amplified barcoded first subfragments, wherein the amplified barcoded first subfragments are double-stranded or partially double-stranded;
(e) introducing staggered single-strand breaks into some of the amplified barcoded first subfragments to generate second complexes, each comprising a plurality of second subfragments; and
(f) associating second capture oligonucleotide sequences with at least some of the second subfragments;

wherein the associating comprises combining the amplified barcoded first subfragments in (d) or the second complexes in (e) with a plurality of individual second beads, wherein each individual second bead comprises a plurality of second capture oligonucleotides immobilized thereon, wherein each second capture oligonucleotide comprises a second capture oligonucleotide sequence, wherein the second capture oligonucleotides immobilized on each individual second bead comprises the same second capture oligonucleotide sequence, wherein a majority of different second beads have different second capture oligonucleotides immobilized thereon, and wherein each different second capture oligonucleotide sequence comprises a different second barcode,
thereby providing a library of barcoded second subfragments.

Methods not encompassed by the wording of the claims but are considered as useful for understanding the invention include a method for preparing a library of barcoded polynucleotides for determining a sequence of a target nucleic acid(s) comprising
(a) providing fragments derived from the target nucleic acid, wherein the fragments are double-stranded or partially double-stranded;
(b) introducing staggered single-stranded breaks into at least some double-stranded fragments, thereby producing a plurality of first complexes, wherein each first complex comprises a plurality of first subfragments, and
(c) associating first capture oligonucleotide sequences with at least some of the first subfragments,
   wherein each first capture oligonucleotide sequence comprises a first barcode, and optionally comprises a promoter sequence or a primer binding sequence, and
   wherein the associating comprises combining the double-stranded fragments in (a) or the complexes in (b) with a plurality of individual first beads, wherein each individual first bead comprises a plurality of first capture oligonucleotides immobilized thereon, wherein each capture oligonucleotides comprises a first capture oligonucleotide sequence, wherein the first capture oligonucleotides immobilized on each individual first bead comprises the same first capture oligonucleotide sequence, wherein a majority of different first beads have different first capture oligonucleotides immobilized thereon, and wherein each different first capture oligonucleotide sequence comprises a different first barcode,
   thereby providing barcoded first subfragments;
(d) amplifying at least a portion of the barcoded first subfragments to produce amplified barcoded first subfragments, wherein the amplified barcoded first subfragments are double-stranded or partially double-stranded;
(e) introducing staggered single-strand breaks into some of the amplified barcoded first subfragments to generate second complexes, each comprising a plurality of second subfragments; and
(f) associating second capture oligonucleotide sequences with at least some of the second subfragments;

wherein the associating comprises combining the amplified barcoded first subfragments in (d) or the second complexes in (e) with a plurality of individual second beads, wherein each individual second bead comprises a plurality of second capture oligonucleotides immobilized thereon, wherein each second capture oligonucleotide comprises a second capture oligonucleotide sequence, wherein the second capture oligonucleotides immobilized on each individual second bead comprises the same second capture oligonucleotide sequence, wherein a majority of different second beads have different second capture oligonucleotides immobilized thereon, and wherein each different second capture oligonucleotide sequence comprises a different second barcode,
thereby providing a library of barcoded second subfragments.

The average length of the first subfragments may be at least 2x greater than the average length of second subfragments in size.

Step (c) may be performed in a single mixture, wherein the number of first beads is greater than the number of target nucleic acid fragments in the single mixture, and wherein each first bead comprises multiple copies of the first capture oligo, immobilized thereon.

First insertion oligonucleotides may be added by ligation or by synthesis to at least some first subfragments in step (b), and wherein step (c) further comprises:
(1) ligating the first capture oligonucleotides to the first insertion oligonucleotides, or
(2) hybridizing first capture oligonucleotides to the first insertion oligonucleotides and then extending the insertion oligonucleotides by a DNA polymerase to incorporate first barcodes.

Step (f) may be performed in a single mixture and wherein the number of second beads is greater than the number of the amplified barcoded first subfragments in the single mixture, wherein each second bead comprises multiple copies of the second capture oligo, immobilized thereon.

Each of at least some second subfragments may be linked to a second insertion oligonucleotide, and wherein step (e) may further comprise:
(1) ligating the second capture oligonucleotide to the second insertion oligonucleotide, or
(2) hybridizing the second capture oligonucleotide to the second insertion oligonucleotide and then extending the insertion oligonucleotide by a DNA polymerase to incorporate second barcodes.

The first insertion oligonucleotides may be introduced by transposition.

The second insertion oligonucleotides may be introduced by transposition

Step (c) may further comprise adding a first splint oligonucleotide to the reaction, wherein the first insertion oligonucleotide comprises a first hybridization sequence that is a complimentary to a first portion of the first splint oligo, and wherein the capture oligonucleotide comprises a common sequence that is complementary to the second portion of the first splint oligo.

Step (f) may further comprise adding a second split oligonucleotide to the reaction, the second insertion oligonucleotide comprises a second hybridization sequence that is a complimentary to a first portion of the second splint oligo, and wherein the capture oligonucleotide comprises a common sequence that is complementary to the second portion of the second splint oligo

The first insertion oligonucleotide may be hybridized to a complementary oligo to form a partially double-stranded first insertion oligonucleotide, and
the first insertion oligonucleotide may be ligated to at least some of the breaks by 3' branch ligation.

Each of the first capture oligo, or each of the second capture oligo, or both, may comprise unique molecular identifiers (UMIs).

Methods not encompassed by the wording of the claims but are considered as useful for understanding the invention include the method wherein:
each first bead comprises multiple copies of the first capture oligonucleotide, wherein the first capture oligonucleotide is hybridized to a complementary oligonucleotide to form a partially double-stranded first capture oligonucleotide,
wherein the method step (b) comprises
ligating the first capture oligonucleotide to each of at least some of the first subfragments by 3' branch ligation, or
ligating the complementary oligonucleotide to each of at least some of the first subfragments by 3' branch ligation, and the extending the complementary oligonucleotide to incorporate the first barcode sequence.

The first capture oligonucleotides may further comprise a promoter sequence, and wherein the amplifying at least a portion of the barcoded first subfragments in step (d) is by
(1) transcribing the barcoded first complexes to generate RNA transcripts,
(2) reversely transcribing the RNA transcripts using a primer annealing to the promoter sequence to generate cDNA strands of the barcoded first complexes,
(3) circularizing the cDNA to produce circularized cDNA strands
(4) amplifying the circularized cDNA strands by rolling circle amplification, and
(5) synthesizing double-stranded or partially double-stranded, barcoded first complexes using the amplified cDNA strands as templates.

The amplifying at least a portion of the barcoded first subfragments may be by
(1) releasing the barcoded first subfragments from first beads,
(2) denaturing barcoded first subfragments that have been released to form single-stranded barcoded first complexes,
(3) circularizing the single-stranded barcoded first subfragments,
(4) performing rolling circle amplification, and
(5) synthesizing double-stranded, barcoded first subfragments using the amplified single-stranded barcoded first complexes as templates.

The method may comprise fractioning the circularized cDNA strands or the single-stranded barcoded first subfragments to select circles of sizes within a predetermined range.

Amplifying at least a portion of the barcoded first subfragments may comprise
(1) extending the barcoded first subfragments using a primer binding to the primer binding sequence on the first capture oligonucleotide,
(2) releasing the extended barcoded first subfragments from (1) from the first beads,
(3) amplifying the barcoded first subfragments by about 10-120 fold using single primer amplification, thereby producing amplified double-stranded barcoded first subfragments, and
(4) ligating an adaptor oligonucleotides to the ends of the amplified double-stranded barcoded first subfragments.

In some approaches, each of at least some second subfragments is linked to a second insertion oligonucleotide, and wherein step (e) further comprises:
(1) ligating the second capture oligonucleotide to the second insertion oligonucleotide, or
(2) hybridizing the second capture oligonucleotide to the second insertion oligonucleotide and then extending the insertion oligonucleotide by a DNA polymerase to incorporate second barcodes
wherein the adaptor oligonucleotides has the same sequence as the second insertion oligonucleotide.

The size of the target nucleic acid fragment may be in the range of 10 kb-100 mega bases.

The size of the first subfragments may be in the range of 1 kb - 20 kb.

Methods not encompassed by the wording of the claims but are considered as useful for understanding the invention include a method for analyzing transcripts comprising:
(a) in a single mixture, combining mRNAs from one or more cells with a population of first beads, wherein each first bead comprises first capture oligonucleotides immobilized thereon, wherein the first capture oligonucleotides comprises a common primer sequence, a first barcode sequence, a UMI, and an oligonucleotides dT sequence, wherein the number of first beads is greater than the number of mRNA molecules in the single mixture,
(b) reverse transcribing the captured RNAs, in the presence of an adaptor template comprising a tri-nucleotide GGG and the common primer sequence, to produce cDNA/mRNA hybrid molecules,
   wherein the cDNA/mRNA hybrid molecules each comprises a cDNA produced from reverse transcription of a captured RNA and the first capture oligo,
(c) releasing the cDNA from the first beads,
(d) amplifying the cDNAs from (c) and producing double-stranded or partially double-stranded cDNAs;
(e) introducing staggered-single strand breaks to at least some of the amplified cDNAs to generate second complexes each comprising a plurality of second subfragments, and
(f) introducing a second capture oligonucleotide to the plurality of second subfragments,
wherein each second capture oligonucleotides comprises
(1) optionally a promoter sequence or a primer binding sequence, and
(2) a second barcode, wherein second capture oligonucleotides immobilized on the same individual bead comprise the same second barcode, and a majority of beads have different second barcodes, thereby providing barcoded second subfragments.

The mRNAs in the single mixture may be from a single cell.

The amplifying the s cDNAs in step (d) may be by rolling circle amplification using a primer hybridized to the common primer sequence.

In some approaches, the each of at least some second subfragments is linked to a second insertion oligonucleotide, and wherein step (f) further comprises:
(1) ligating the second capture oligonucleotide to the second insertion oligonucleotide, or
(2) hybridizing the second capture oligonucleotide to the second insertion oligonucleotide and then extending the insertion oligonucleotide by a DNA polymerase to incorporate second barcode

The amplifying the cDNAs in step (d) may comprise amplifying the cDNAs by single primer amplification using a primer hybridized to the common primer sequence, and ligating an adaptor oligonucleotides to the ends of the amplified first complexes, wherein the adaptor oligonucleotides has the same sequence as the insertion oligonucleotide.

Each of the first insertion oligonucleotides may comprise a first positional barcode, wherein different first insertion oligonucleotides comprises different first positional barcodes, and/or
wherein each of the second insertion oligonucleotides may comprise a second positional barcode, wherein different second insertion oligonucleotides comprises different second positional barcodes.

One or more first positional barcodes may be the same as one or more second positional barcodes.

Methods not encompassed by the wording of the claims but are considered as useful for understanding the invention include a method of analyzing the full-length sequence of one or more target region comprising
(a) amplifying each target region,
(b) ligating an adaptor oligonucleotide to both ends of amplified nucleic acid target fragments comprising the target region,
(c) introducing staggering single-stranded breaks in at least some of the amplified nuceic acid fragments from (b), to produce a plurality of first complexes each comprising a plurality of first subfragments,
(d) introducing first capture oligonucleotides to at least some of the first subfragments, wherein each first capture oligonucleotides comprises
   (1) optionally a promoter sequence or a primer binding sequence, and
   (2) a first barcode, wherein first capture oligonucleotides immobilized on the same individual bead comprise the same first barcode, and a majority of beads have different first barcodes, thereby providing barcoded first subfragments.

Amplifying the target region in step (a) may comprise:

Amplifying the target region with a forward primer and a reverse primer, both comprising sequences specific to the target region, thereby producing an amplified region,
wherein forward primer comprises a common sequence and a UMI, and a first target specific sequence, and wherein the forward primer comprises special bases, wherein the special bases are not present in native DNA molecules and the special bases can be degraded by an agent;
degrading the excessive forward primers by adding the agent
amplifying the amplified region in (i) using a primer binding to the common sequence and the reverse primer, thereby producing further amplified region comprising the UMI.

The special bases may be uracils and the agent may be capable of specifically cleaving uracil-containing oligonucleotides.

Amplifying the target region in step (a) may further comprise
(4) ligating two oligonucleotides, each to one end of the further amplified region from (3), wherein the two oligonucleotides share the same common sequence, and wherein the two oligonucleotides comprise different UMIs, thereby producing a ligated DNA product having common sequences at both ends, and
(5) amplifying the ligated DNA product from (4) with primers hybridized to the common sequences, thereby producing amplified nucleic acid target fragments comprising the target region.

The method may further comprise ligating a 3' branch ligation adaptor oligonucleotide to the second subfragments, wherein the ligating an adaptor oligonucleotide is a 3' branch ligation, and wherein the adaptor oligonucleotide comprises a second PCR primer annealing site.

The 3' branch ligation adaptor oligonucleotide may be a blunt end adaptor and the 3' branch ligation may comprise the covalent joining of the 5' phosphate from the blunt-end adapter to the recessed 3' hydroxyl a the nicks of the first fragments.

Tthe first PCR primer annealing site and the second PCR primer annealing site may have different sequences.

The the 3' branch ligation adaptor oligonucleotide may comprise a barcode sequence, with is optionally a sample barcode sequence.

When introducing first insertion oligonucleotides, introducing the second insertion oligonucleotides, or both, may be by transposase, and the insertion oligonucleotidetransposase remains bound to the first subfragments or insertion oligonucleotide remain bound to the second subfragments, or both.

the method may comprise removing the transposase thereby separating individual first subfragments, second subfragments, or both.

The method may further comprise amplifying the first subfragments, second subfragments, or both, to produce amplicons using primers annealing to the first and second PCR primer annealing sites.

At least some of the first insertion oligonucleotidesmay each comprise a positional barcode, wherein different first insertion oligonucleotides comprise different positional barcodes.

At least some of the second insertion oligonucleotides may each comprise a positional barcode, wherein different second insertion oligonucleotides comprise different positional barcodes.

A method for preparing a sequencing library for sequencing a target nucleic acid comprising:
(a) providing single-stranded DNA circles of the target nucleic acid and a plurality of first beads,
   wherein each first bead comprises a plurality of first capture oligonucleotides,
   wherein each first capture oligonucleotide comprises a first barcode comprising a first barcode sequence,
   wherein all first capture oligonucleotides immobilized on the same individual bead comprise the same first barcode sequence, and a majority of beads have different first barcode sequences,
(b) amplifying the single-stranded DNA circles from (a) by rolling circle amplification to produce a plurality of single-stranded concatemers that comprise at least five copies of a single-stranded fragment,
(c) converting the single-stranded concatemers into double-stranded or partially double-stranded DNA molecules,
(d) introducing staggered single-stranded breaks to the DNA molecules in (c), thereby generating first complexes, each comprising a plurality of first subfragments from one of the DNA molecules in (c),
(e) associating at least some of the first subfragments in the first complexes with first capture oligonucleotides immobilized on a plurality of individual first beads, wherein each individual first bead comprises a plurality of the first capture oligonucleotides, thereby providing barcoded first subfragments.
In some approaches, each of at least some first subfragments is linked to a first insertion oligonucleotide, and wherein step (e) further comprises:
(1) ligating the first capture oligonucleotide to the first insertion oligonucleotide, or
(2) hybridizing the first capture oligonucleotide to the first insertion oligonucleotide and then extending the insertion oligonucleotide by a DNA polymerase to incorporate first barcode.

In some approaches, each of the insertion oligonucleotides comprises a positional barcode, wherein different insertion oligonucleotides comprises different positional barcodes.

Methods not encompassed by the wording of the claims but are considered as useful for understanding the invention include a method of inserting oligonucleotides into fragments of a target nucleic acid comprising:
(a) introducing staggered single-stranded breaks into the fragments,
(b) contacting the fragments from (a) with an insertion scaffold, wherein the adaptors are anchored to the scaffold and separated by predetermined spacing, wherein the insertion scaffold comprises a plurality of double-stranded or partially double-stranded adaptors and a scaffold,

wherein each adaptor comprises an insertion oligonucleotide comprising a unique positional barcode, and
wherein the contacting results in the plurality of insertion oligonucleotides being introduced into the fragments at the single-stranded breaks, thereby producing first insertion complexes, each comprising a plurality of first subfragments.

The method may further comprise:
dissociating the scaffold from the plurality of adaptors that have been inserted into the target nucleic acid.

The method may comprise
contacting a plurality of scaffolds with each of some of the nucleic acid fragments, wherein adaptors in different scaffolds have different scaffold barcodes.

The scaffold may be a single-stranded nucleic acid molecule, and
wherein each adaptor may further comprise
   a scaffold hybridization sequence, wherein the transposon hybridizes to the scaffold via the scaffold hybridization sequence, and
wherein the scaffold hybridization sequence can be cleaved to dissociate the scaffold from the adaptor.
wherein the insertion oligonucleotide further comprises a scaffold barcode shared by all adaptors within the scaffold.

The method may further comprise:
(c) combining in a single mixture (i) the first insertion complexes produced from (a); and (ii) a population of first beads, wherein each first bead comprises multiple copies of a first capture oligonucleotides immobilized hereon, said first capture oligonucleotides comprising a first barcode, wherein the first capture oligonucleotides immobilized on the same individual first bead comprise the same first barcode and a majority of beads have different first barcodes,
(d) for each of a plurality of the first subfragments, introducing the first capture oligonucleotide, thereby producing barcoded first subfragments each is linked to a copy of the first barcode.

In some approaches, the method further comprise:
(e) amplifying the plurality of barcoded first subfragments,
(f) introducing staggered single-stranded breaks to the amplified barcoded subfragments, and
(g) contacting with the products from step (f) with second insertion scaffolds, wherein the second insertion scaffolds each comprises a plurality of second adaptors anchored to a second insertion scaffold, thereby introducing second insertion oligonucleotides on the second adaptors to the amplified barcoded first subfragments to produce second insertion complexes, each comprising a pluratlity of second subfragments.

The scaffold of the second insertion scaffold may be a single-stranded nucleic acid molecule, and each of the second adaptor may comprise:
(1) a second scaffold hybridization sequence,
   wherein the second adaptor hybridizes to the scaffold via the second scaffold hybridization sequence, and
   wherein the second insertion scaffold hybridization sequence can be cleaved to dissociate the scaffold from the second adaptor;
(2) a second insertion oligonucleotide comprising a unique positional barcode, and a second scaffold barcode shared by the second adaptors within the scaffold of the second insertion scaffold.

The second insertion complexes may be mixed with a population of second beads, wherein each bead comprises second capture oligonucleotides immobilized hereon, said oligo nucleotides comprising a second barcode, wherein the oligonucleotides immobilized on the same individual bead comprise the same second barcode and a majority of beads have different second barcodes,
(g) for each of at least some of the second insertion complexes, introducing multiple copies of the second capture oligonucleotide comprising a second barcode, wherein the multiple copies are from a single bead, and thereby producing a plurality of barcoded second subfragments, each is linked to at least one copy of the second barcode.

The method may further comprises:
sequencing the plurality of second subfragments to produce a number of sequencing reads.

The insertion scaffold may have a size of 1-50kb.

The predetermined intervals between the adjacent first adaptors may range from 3 kb to 5 kb.

Predetermined intervals between the adjacent second adaptors may range from 200 bp to 1000 bp.

The sum of the lengths of the plurality of insertion scaffolds may be equal to or greater than the length of the target nucleic acid.

Examples not encompassed by the wording of the claims but are considered as useful for understanding the invention include a plurality of insertion scaffolds,
wherein each of a plurality of insertion scaffolds comprises
   (1) a plurality of adaptors, wherein the adaptors are double-stranded or partially double-stranded, and
   (2) a scaffold, and the adaptors are anchored to the scaffold and separated by predetermined spacing,
wherein for each insertion scaffold, each adaptor in the insertion scaffold carries a unique positional barcode and a common scaffold barcode, and
wherein adaptors in different insertion scaffolds have different scaffold barcodes.

Aat least one of the insertion scaffolds may have a size that is equivalent to the size of a polynucleotide of 1-10kb.

At least one of the insertion scaffold may have a size that is equivalent to the size of a polynucleotide of 10-50kb.

Examples not encompassed by the wording of the claims but are considered as useful for understanding the invention include a nucleic acid complex comprising a plurality of insertion scaffolds disclosed herein, and a nuclear acid fragment, wherein the plurality of insertion scaffolds are hybridized to the target nuclear acid fragment.

Examples not encompassed by the wording of the claims but are considered as useful for understanding the invention include a reaction mixture that is in a single vessel, wherein the reaction mixture comprises a plurality of insertion scaffolds disclosed herein and multiple fragments derived from a target nucleic acid.

### BRIEF DESCRIPTION OF THE DRAWINGS

The drawings and descriptions thereof illustrate examplary embodiments of the invention. The inventions provided in this disclosure are not limited to the embodiments shown in these drawings.
FIG. 1 shows electrophoresis of genomic DNA fragments generated by Tn5 insertions.

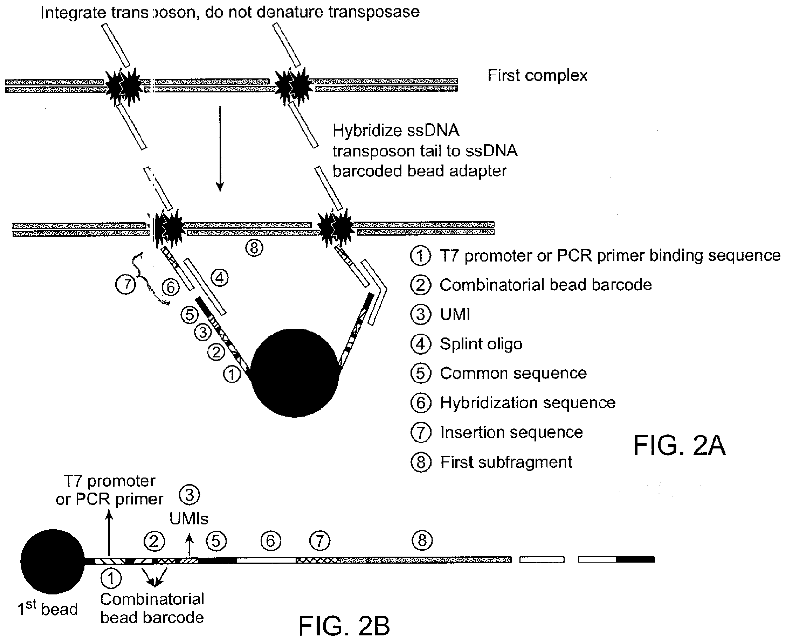

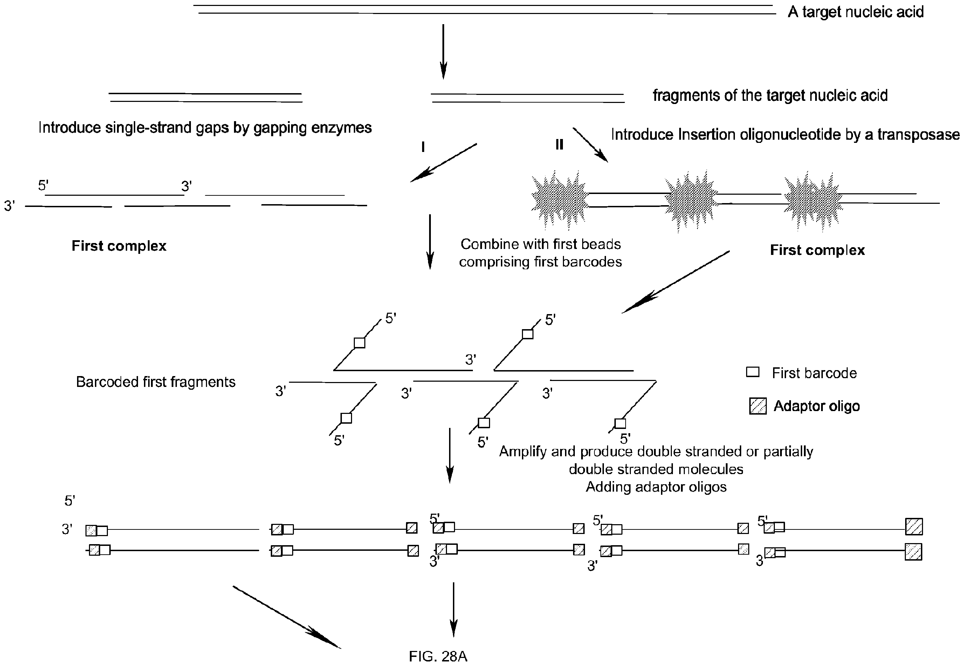

FIG. 2A shows an exemplary method of capturing the DNA fragments on beads that comprise immobilized capture oligonucleotides. Transposons comprising insertion oligonucleotides ("first insertion oligonucleotides") are first integrated into a double-stranded or partially double-stranded target nucleic acid fragment by a transposase to form first complexes. The transposases nick the target nucleic acid but remain attached to the target nucleic acid. The resulted fragments ("first fragment"} were incubated with beads, each bead comprising multiple capture oligonucleotides (only two of which are shown in FIG. 2A). Each capture oligonucleotides comprises a T7 promoter or PCR primer binding sequence ①, a unique barcode sequence ② ("combinatorial bead barcode"), unique molecular identifiers (UMI) ③, and a common sequence ⑤. Each transposon comprises a hybridization sequence ⑥ an insertion oligonucleotide ⑦,which comprises hybridization sequence ⑥. The capture oligonucleotides on the same bead share the same unique barcode sequence. A splint oligonucleotide ④comprises a portion that is hybridized to a common sequence ⑤ and a portion that is hybridized to a hybridization sequence ⑥. The portion of the first complex between a one transposon and a flanking transposon is referred to as a first subfragment ⑧. As used herein, a "flanking transposon" refers to the transposon closest to the reference transposon. If the reference transposon has two flanking transposons (one upstream and one downstream) the both are flanking transposons, and also referred to as to adjacent transposons. As used in this disclosure, a complex, as used in "a first complex" or a "second complex", as well as "first insertion complex" or "second insertion complex" refers to a series of single oligonucleotide strands that are interconnected to form a partially double-stranded DNA structure having staggered single-stranded breaks. In some cases, these single oligonucleotide strands are interconnected through enzymes that bind to the termini of these strands, for example, transposase molecules, as shown in FIG. 2A. In some cases, these single oligonucleotide strands are interconnected through overlapping regions as shown in FIG. 28A ("I"). Thus, depending on the context, in some embodiments, the first complex may further comprise enzymes (transposase) and/or insertion oligonucleotides inserted at regular intervals. see FIG. 28A ("II"). As disclosed in this disclosure, a subfragment, as used in "a first subfragment" or "a second subfragment", refer to as a single oligonucleotide strand, which is part of double-stranded or partially double-stranded DNA. FIG. 2B shows the DNA molecule formed after ligating the capture oligonucleotides with the insertion oligonucleotide in the transposon.
FIG. 3 shows an illustrative embodiment of the invention in which capture oligonucleotides are ligated to the inserted genomic fragments and the fragments are extended through nick translation such that one capture oligonucleotide is added to each end of each of the first subfragments ⑧. In vitro transcription is performed to amplify the newly formed DNA fragments in a linear fashion.
FIG. 4 shows an illustrative embodiment of the invention in which performing reverse transcription on the transcripts generated from method steps in FIG. 3 with a primer annealed to the T7 promoter region to produce cDNA. Second strands are subsequently synthesized using primers binding to the common sequence to produce double-stranded DNA molecules.
FIG. 5 shows, as an alternative to the approach in FIG. 3, in which the fragments produced from FIG. 2B are extended by nick translation and the extended fragments are released from the beads. Single primer long range PCRs is performed using a primer that binds to the PCR primer binding sequence ① to produce double-stranded DNAs.
FIG. 6A and 6B show an approach in which adaptor oligonucleotides ⑨ are ligated to the ends of the double-stranded DNA molecules as depicted in FIG. 4 and FIG. 5, respectively.

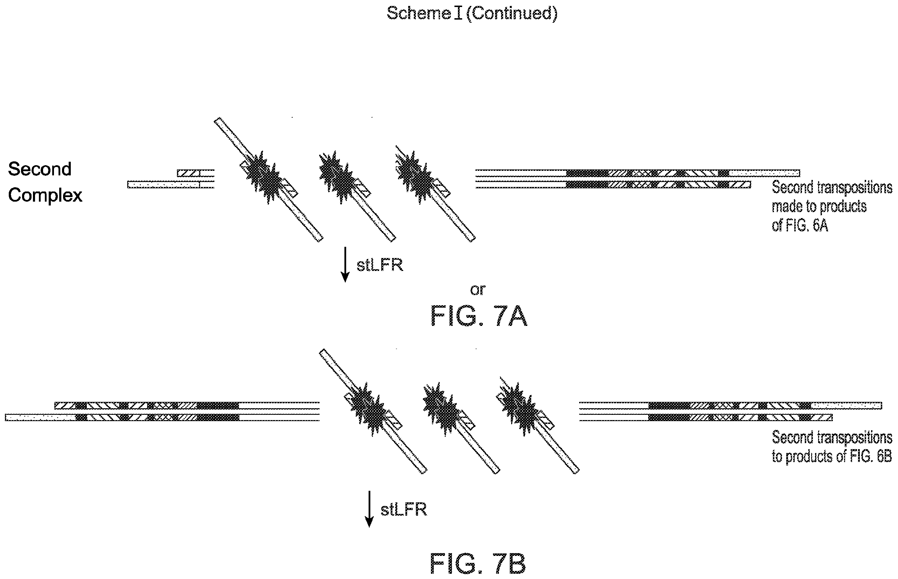

FIG. 7A and 7B show an approach in which insertion oligonucleotides ("second insertion oligonucleotides") are introduced by transposons into the double-stranded DNAs ligated with adaptor oligonucleotides produced from steps in FIG. 6A and 6B, respectively. The second insertion oligonucleotide has the same sequence as the adaptor oligonucleotides ⑨ as depicted in FIG. 6A and 6B.
FIG. 8 shows an approach in which full length mRNA is captured on beads that are immobilized with unique bead barcode ②, random molecule barcode (UMI) ③, common primer binding sequence ①, and oligonucleotides dT (10).
FIG. 9 shows an approach involving reverse transcribing and incorporating an adaptor oligonucleotide (13) into the cDNA molecule (11), via the tri-nucleotide CCC tail added by an RT enzyme and an adapter template (12) comprising the tri-nucleotide GGG.
FIG. 10 shows an approach involving releasing molecules from beads and performing PCR with a common primer that recognizes the common primer binding sequence ①. The fold of amplification can be 10 to 1 million or more, depending on the desired coverage of each transcript required.
FIG. 11 shows an approach in which adaptor oligonucleotides ⑨ are ligated to the ends of the dsDNA molecules produced from the method steps depicted in FIG. 10.
FIG. 12 shows an approach involving transposing insertion oligonucleotides into the dsDNA molecules depicted in FIG. 11. The dsDNA molecules have the adaptor oligonucleotides at both ends. The insertion oligonucleotides have the same sequence as the adaptor oligonucleotides ⑨.

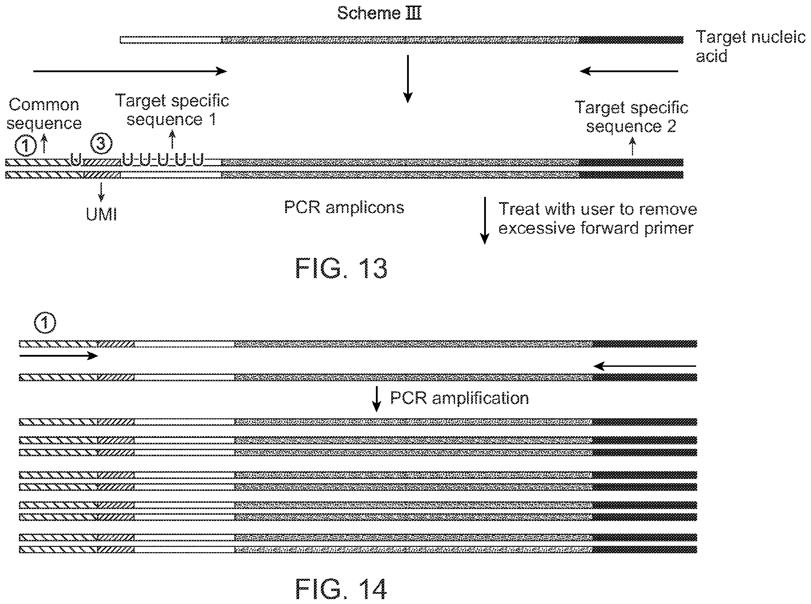

FIG. 13 shows an approach in which an amplified PCR product is produced by amplifying a target region using a forward and a reverse primer. The forward primer comprises a target specific sequence that is complementary to a first sequence ("Target specific sequence 1") in the target region and the reverse primer comprises a target specific sequence that is complementary to a second sequence ("Target specific sequence 2") in the target region. The forward primer further comprises a UMI ③ and a common sequence ①. The target specific sequence in the first primer comprises a plurality of uracils in place of thymidines.
FIG. 14 shows an approach in which the reaction mixture is treated with the enzyme Uracil-Specific Excision Reagent (USER) to remove excessive forward primer, and the target region is further amplified using a forward primer that binds to the common sequence ① and the reverse primer that binds to the second sequence in the target region.
FIG. 15 shows an approach in which adaptor oligonucleotides ⑨ are ligated to both ends of the amplification products in FIG. 14.
FIG. 16 shows an approach involving amplifying the target region as an alternative to the method depicted in FIG. 13. The method in FIG. 16 amplifies the target region using a forward primer and a reverse primer as in FIG. 13. The target region is amplified for a limited number of PCR cycles.
FIG. 17 shows an approach involving ligating a common sequence oligonucleotide (14) to the each end of the amplified products produced from method steps in FIG. 16. Each common sequence oligonucleotide comprises a common sequence at the 5' end and a UMI at the 3' end.
FIG. 18 shows an approach involving amplifying the ligation product from FIG. 17 with a single primer annealing to the common sequences. The amplified ligation product is ligated with adaptor oligonucleotides at both ends.
FIG. 19 shows an approach involving transposing insertion oligonucleotides into the product obtained from method steps in FIG. 18 and the insertion oligonucleotides have the same sequence as the adaptor oligonucleotide.
FIG. 20 shows , as an illustrative embodiment of the invention, a barcode sequence assembly. Three ligations are used necessary to generate ~3.6 billion different barcodes. The expected sequence at each step of the barcode assembly is displayed.
FIG. 21 shows an illustrative embodiment of procedures for the barcode sequence assembly.
FIG. 22A shows, as an illustrative embodiment of the invention, circularizing a single-stranded DNA . FIG. 22B shows performing rolling circle amplification ("RCR") on the circle. FIG. 22C shows converting the amplification product from the RCR reactions into double-stranded DNA, or partially double-stranded DNA (not shown). In some embodiments, the amplification products can also comprise branched structures (not shown). FIG. 22D shows insertion of transposons into the double-stranded DNA.

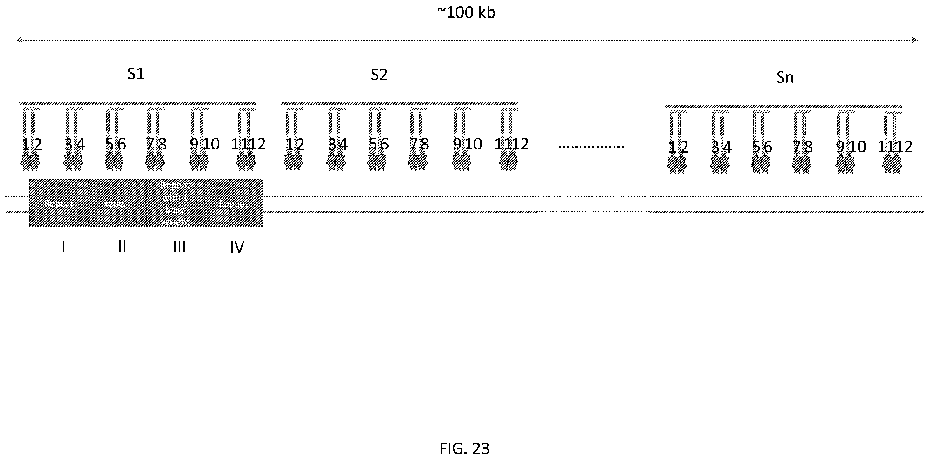

FIG. 23 illustrates an approach involving using insertion scaffolds S1, S2, ..., Sn to transpose target nucleic acids. Each of the adaptors, e.g., transposon elements, comprise two positional barcodes (e.g., 1 and 2, 3 and 4, etc.) and a scaffold barcode (the vertical bars above the numbers referencing the positional barcodes), which is unique to the scaffold. FIG. 23 illustrates that in repetitive regions, individual transposon barcodes can help with ordering of repeat sequences. 1-50 kb scaffold barcodes can help with longer range repeat regions. Fixed spacing between adjacent transposons and scaffold barcodes can help determine the size of the repetitive regions. The transposons shown in FIG. 23-25B can also be replaced by any double-stranded or partially double-stranded adaptors to perform the methods disclosed herein.
FIG. 24 shows the configuration of an exemplary transposon scaffold. The transposons are hybridized to the scaffold via scaffold hybridization sequence, thus fixing the order of transposons along the scaffold.
FIG. 25A illustrates a first round of insertion with transposons with positional barcodes. In this example, the transposon results in 3-5 kb sized molecules. These molecules are captured on stLFR beads and co-barcoded (not shown), circularized, amplified by RCR, and converted to double-stranded or partially double-stranded DNA molecules.

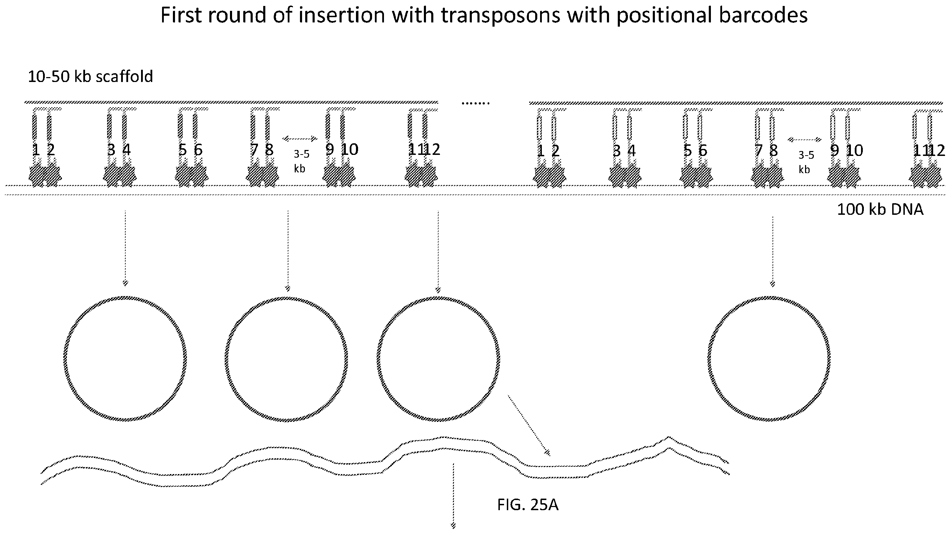

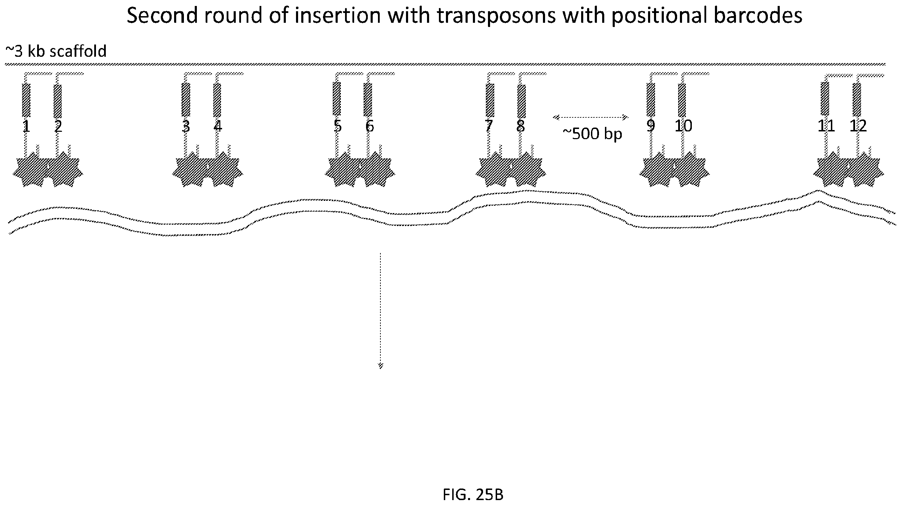

FIG. 25B illustrates the process of performing a second round of insertion after the first round of insertion in FIG. 25A. The double-stranded DNA molecules produced in the process shown in FIG. 25A can be transposed again using a second transposon scaffold with transposons anchored thereon at a shorter spacing. The second round of transposition results in about 500 bp between adjacent transposons. Analysis of barcodes introduced by stLFR beads can result in positional information from the first round (resulting in first subfragments of 3-5 kb) and the second round transpositions (resulting second subfragments of about 500bp) . In addition, insertion of transposons results in a 9 bp overlap between adjacent sub-fragments, this information can also be used to order subfragments between scaffolds. (We can also perform this strategy through ligation of adapters to gapped DNA. We don't want to be specific only to transposon insertion.)
FIG. 26 shows approach involving constructing sequence information for a 100 kb genomic DNA molecule using layers of positional barcodes and the 9 bp overlap resulted from each insertion of the transposons. A first round of transposition is performed using transposon scaffold having a length that ranges from 10 to 50 kb. A second round of transposition is performed using transposon scaffolds having a length that ranges from 3-5kb.
FIG. 27 A and 27B show an approach in which nicks are first generated in target the fragments by a nickase. The nicks are widened using Klenow fragment or any other enzyme with 3'-5' exonuclease activity to form single-stranded gaps. The resulted DNA fragments are ligated to partially double-stranded capture oligonucleotide adaptors on the beads by 3' branch ligation. In FIG. 27A, the adaptor strand complementary to the capture oligonucleotide is ligated to the DNA fragments. In FIG. 27B, the adaptor strand comprising the capture oligonucleotide is ligated to the 3' end of the DNA fragments

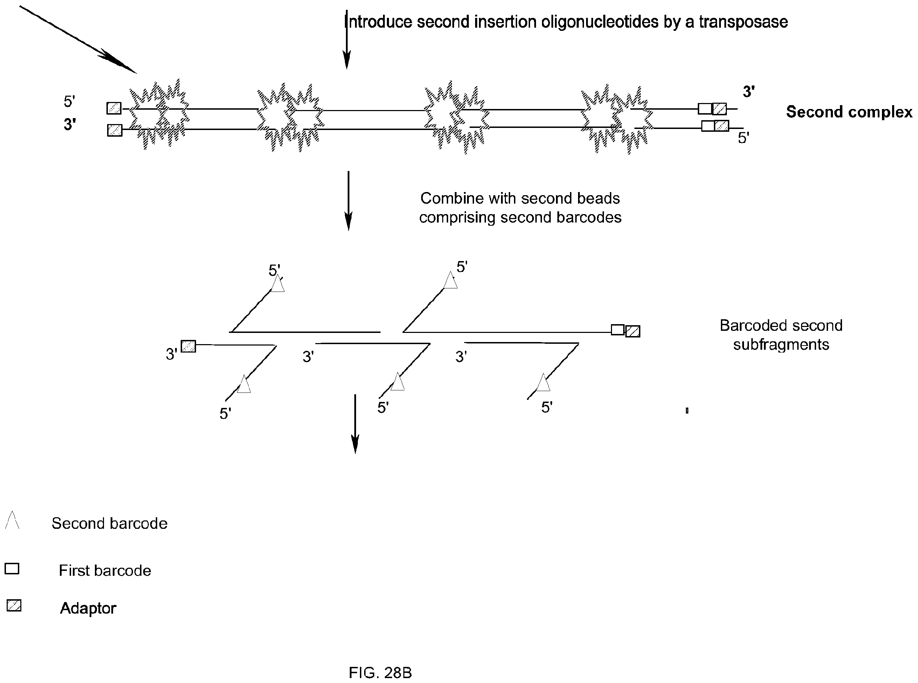

FIG. 28A and FIG. 28B show embodiments according to scheme I of the disclosure. FIG. 28A shows the first round of stLFR in which a target nucleic acid is fragmented to produce multiple double stranded target nucleic acid fragments. In one approach ("I"), staggered single stranded breaks are introduced into the fragments. In an alternative approach ("II"), staggered singe stranded breaks (nicks) are introduced by transposase/transposon. Although only one first complex produced from either approach is shown, it is appreciated that multiple fragments are processed in the same manner and will produce multiple first complexes. The first complexes each comprise a plurality of first subfragments. The first complexes are combined with first beads having multiple copies of first capture oligonucleotides comprising first barcodes. As described in the disclosure, the first barcode sequence is introduced into each of the first subfragments to produce a plurality of barcoded first subfragments. These barcoded first subfragments are amplified, resulting double-stranded or partially double-stranded first subfragments. Adaptor oligonucleotides are then added to both ends. FIG. 28B shows the second round of stLFR, in which the double stranded first subfragments are transposed with transposons carrying second insertion oligonucleotides (insertion oligonucleotides are not shown in this figure). Similar to the first round stLFR in the FIG. 28A, the step can also be performed using gapping enzymes and adaptors to introduce insertion oligonucleotides. The second complexes (only one is shown) each comprising a plurality of second subfragments are combined with second beads carrying second barcodes, which results in the second barcode sequences being added to the second subfragments, producing barcoded second subfragments.

### DETAILED DESCRIPTION

### I. Overview

The first generation of stLFR uses the surface of a microbead as a replacement for a compartment (e.g., the well of a 384 well plate) that is used in conventional barcoding reactions. Each bead carries many copies of a unique barcode sequence which is transferred to the subfragments of each long DNA molecule. Because each long DNA molecule carries multiple copies of the same barcode, the process is referred to as "co-barcoding". The first generation of stLFR thus allows construction of sequence information of long DNA fragments based on sequencing reads of co-barcoded subfragments generated in a single tube. The first generation of stLFR is described in the co-pending PCT Patent Publication No. WO 2019/217452.

Here we describe a high coverage single tube Long Fragment Read (stLFR) technology which uses a combination of multiple barcodes and UMIs to allow sequence construction that covers more of the long DNA fragments as compared to the first generation stLFR. This technology performs stLFR on target DNA fragments that have already been amplified before they are co-barcoded, which provides higher amount of DNA for sequencing and increases sequencing coverage. In some embodiments, the high coverage stLFR described herein uses two rounds of stLFR. The first round of stLFR labels a target DNA fragment (e.g., a long genomic DNA fragment) by inserting or ligating many copies of a first barcode that is unique to the target DNA fragment using the first beads. In some embodiments, a UMI is also added with the first barcode to the target DNA fragment. The addition is infrequent so that each of the first subfragments generated (located between the two adjacent first barcodes) is relatively large, e.g., 10 kb. The first subfragments are then amplified. Within each first subfragment, a second round of stLFR introduces second barcodes at a frequency that is higher than the first round of labelling to produce second complexes. Due to the higher frequency of labelling, the second subfragments, which are located between the adjacent second barcodes, are smaller than the first subfragments. The second subfragments are of a length that is suitable for certain sequencing methods, for example, about 100 -1000 bases. The sequence reads from the second subfragments can be combined based on the same second barcode to construct the sequence for each of the first subfragments; and the constructed sequence from each of first subfragment can be combined to construct the original target DNA molecule, which can be a very long genomic fragment, e.g., about 40 kb-400 kb. Because of this additional barcoding, this improved stLFR increases the coverage by at least 10 fold and can be used to construct the sequence of very long target nucleic acid molecules.

In some embodiments, the high coverage stLFR can be used to analyze the transcriptome of a single cell. One approach, the cells are diluted in suitable vessels(e.g., droplets or wells) so that most compartments contains only one or zero cell. The cell is lysed and the cell lysate comprising mRNA from the single cell is mixed with beads with a unique first barcode, a UMI, and a dT oligonucleotides to capture mRNAs from the single cell. The mRNA is then reverse transcribed to produce cDNA, and double-stranded or partially double-stranded DNA molecules are produced from the cDNA using routine methods. The amplified double-stranded DNA is then subjected to stLFR, which adds many copies of unique second barcodes. In these embodiments, the sequence of the transcript can be assigned to individual cells based on the first barcode (each first barcode corresponds to a single cell) and sequence can be assigned to each mRNA based on the UMI. The second barcode is used to construct sequence from each long transcript by combining sequence reads having the same second barcode.

In some embodiments, the high coverage stLFR uses target specific primers to amplify a target region before performing stLFR. In one approach, PCR is used to amplify the target region. The PCR primers comprise a common sequence and one or both PCR primers may comprise a UMI. The presence of UMI enables the user to distinguish errors produced by the PCR process from the mutations in the sequence itself, increasing the confidence of variant calling.

### II. Definitions

The term "staggered single-stranded breaks" refers to breaks (produced by nicking, gapping, and/or deletion of nucleotides) introduced to a DNA molecule (double-stranded or partially double-stranded), resulting in a plurality of single-stranded DNA molecules. For at least some of the single-stranded DNA molecules a portion of the 5' sequence is complementary to at least a portion of the 5' sequence of another single-stranded DNA molecule and at least a portion of the 3' end is complementary to at least a portion of the 3' sequence of yet another single-stranded DNA molecule such that under hybridization conditions a plurality of single-stranded DNA molecules hybridize to each other to form a nucleic acid complex. For illustration and not limitation, a nucleic acid complex comprising three single-stranded DNA molecules having staggered single-stranded breaks is illustrated below. It will be appreciated that a nucleic acid complex (or "complex") may, and typically does, comprise more than three single-stranded DNA molecules.

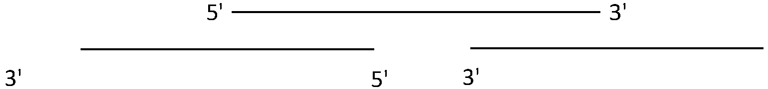
5' 3'

3' 5' 3'

The length of the region of hybridization between a pair of single strand DNA molecules can range from, for example, 20 base pairs to 1000 base pairs or more. The region of non-hybridization (e.g., the unhybridized region of the upper DNA molecule above), if any, can range from, for example, a lower limit of at least 1 base, at least 2 bases, at least 3 bases, at least 5 bases, at least 10 bases, or at least 15 bases, and an upper limit of 20 bases, 100 bases, 1000 bases, or more than 1000 bases. In some cases the region of non-hybridization is zero base (e.g., strand is nicked without deletion of a nucleotide). The term "staggered double-stranded breaks" has the same meaning.

The term "partially double-stranded" refers to two DNA strands that are hybridized to each other and at least a portion of one strand is not hybridized the other strand. The two DNA strands of a partially double-stranded DNA may be of different length or maybe of the same length.

The term "transposon", as used herein, refers to a nucleic acid segment that is recognized by a transposase or an integrase enzyme and is capable of being inserted into a DNA molecule by a transposase.

The term "transposase" as used herein has its usual meaning in the art and refers to an enzyme that that binds to the end of a transposon and catalyzes its insertion into a polynucleotide (e.g., by a cut and paste mechanism or a replicative transposition mechanism)..

As used herein, "unique molecular identifier" (UMI) refers to sequences of nucleotides present in DNA molecules that may be used to distinguish individual DNA molecules from one another. See, e.g., Kivioja, Nature Methods 9, 72-74 (2012). UMls may be sequenced along with the DNA sequences with which they are associated to identify sequence reads that are from the same source nucleic acid. The term "UMI" is used herein to refer to both the nucleotide sequence of the UMI and the physical nucleotides, as will be apparent from context.

UMls may be random, pseudo-random or partially random, or nonrandom nucleotide sequences that are inserted into adapters or otherwise incorporated in source nucleic acid (e.g, DNA) molecules to be sequenced. In some implementations, each UMI is expected to uniquely identify any given source DNA molecule present in a sample.

As used herein, the term "single tube LFR" refers to the process described in, e.g., US patent publication 2014/0323316, in which, *inter alia,* multiple copies of the same, unique barcode sequence are associated with individual long nucleic acid fragments, for example, through ligation. In a typical embodiment of single tube LFR, the long nucleic acid molecule is labeled with "insertion oligonucleotides" at regular intervals. In one embodiment, the insertion oligonucleotides are introduced into the long nucleic acid molecule by one or more enzymes, e.g., transposases. Nickases, ligases. The barcode sequences among different long nucleic acid fragments are different. Thus, the process of labeling individual long nucleic acid fragments can be conveniently performed in a single vessel. This process allows analysis of a large number of individual DNA fragments without the need to separate fragments into separate tubes, vessels, aliquots, wells, or droplets during tagging steps.

As used herein, a "unique" barcode refers to a nucleotide sequence that is associated with, and can be used to distinguish, individual beads. In a population of beads each having a unique barcode, the barcode sequence associated with one bead is different from barcode sequences of at least 90% of the beads in the population, more often at least 99% of the beads in the population, even more often at least 99.5% of the beads in the population, and most often at least 99.9% of the beads in the population.

As used herein the term "adjacent", used interchangeably with "flanking", used in connection with nucleic acid sequences (e.g., as used in, "adjacent barcodes", "adjacent transposons", "adjacent nicks", "adjacent breaks", "adjacent insertion oligonucleotides", etc.), refers to two closest nucleic acid sequences of a series of spatially separated nucleic acid sequences, unless otherwise indicated or clear from context,. As an example, the term "adjacent insertion oligonucleotides" refers to two closest insertion oligonucleotides incorporated into a target nucleic acid fragment during an stLFR process, i.e., there are no insertion oligonucleotides between these two.

The term "link", used in connection with two or more nucleic acid sequences, as used in, e.g., a first subfragment is linked to a first barcode, refers to the two nucleic acid sequences are connected directly or indirectly. The linking can be done by a number of methods well known in the art, e.g., by ligation or by synthesis.

### III. Exemplary embodiments of the methods

The high coverage stLFR can be carried out according to various schemes. The following are exemplary embodiments of the methods and variations to these embodiments. A practitioner with skill in the arts of molecular biology and sequencing guided by this disclosure will recognize numerous variations that can be incorporated into the schemes below.

In a typical embodiment of single tube LFR, staggered single-stranded breaks are introduced to long nucleic acid molecules (e.g., fragments of a target nucleic acid) and insertion oligonucleotides are inserted at breaks. In one embodiment, the insertion oligonucleotides and the staggered single-stranded breaks are introduced by one or more transposons. In another embodiment, the staggered single-stranded breaks are introduced by gapping enzymes (e.g., nickase, klenow), and the insertion oligonucleotides are ligated to the fragment at the staggered single-stranded breaks through a branched ligation reaction. The long nucleic acid molecule with the insertion oligonucleotides is in contact with a bead, on which multiple copies of capture oligonucleotides immobilized thereon and each of the capture oligonucleotides comprises a barcode that is unique to the bead. The barcode sequence can be transferred to the long nucleic acid fragment through a number of ways. In one approach, it is transferred through hybridization among the insertion oligonucleotide, a splint oligo, and a common sequence on the capture oligo, with a portion of the splint oligonucleotides hybridizing to a portion of the insertion oligonucleotide and another portion of the splint oligonucleotides hybridizing to common sequence on the capture oligo. In another approach, the barcode sequence is transferred to the long nucleic acid fragment by hybridization between the capture oligonucleotide and the insertion oligonucleotide. The insertion oligonucleotide is then extended to produce a copy of the barcode sequence.

### Scheme I. Preparation of long nucleic acid fragment for sequencing with greater coverage

Genomic DNA or double-stranded cDNA from a sample is fragmented to generate fragments of target nucleic acid, and at least some of these fragments are then subjected to a first round of stLFR. The first round of stLFR for each target nucleic acid fragment results in a plurality of first subfragments, each of at least some of these first subfragments is linked to a copy of a first barcode ("barcoded first subfragments"). The barcoded first subfragments are amplified and the amplified products are subjected to a second round of stLFR. The second round of stLFR results in second subfragments, each of the at least some of these second subfragments is linked to a copy of a second barcode ("barcoded second subfragments"). At least one of the second subfragments contains both the second barcode and a first barcode. The second subfragments are sequenced and sequence reads from second subfragments having the same second barcode can be assembled together to identify the sequence of the first subfragment having the first barcode. It will be appreciated that the sequence reads in this step will include second barcode sequence. Then, sequences from first subfragments having the same first barcode can be assembled together to produce the sequence information for the target nucleic acid fragments. In this manner, sequences of long DNA fragments can be determined. An illustration of the embodiment is shown in FIG. 28A and 28B. Although described here as a process in which sequence reads are assembled in two steps, with the first step assembly based on first barcoded and the second step assembly based on the second barcodes, it will be appreciated that both sequence assembly steps can be carried out concurrently and/or in multiple iterations. Details of illustrative embodiments of the methods are described as follows.

### 1. First insertions in the first round of stLFR

In one approach, the target nucleic acid fragments are incubated with transposons and transposase. Alternatively, in a related approach, the target nucleic acids are combined with gapping enzymes --enzymes that create staggered single-stranded breaks in double-stranded DNA (e.g., nickases or DNA polymerases provided without free nucleotides)-- along with ligases and adapters comprising the insertion oligonucleotides. This results in the introduction of the insertion oligonucleotide (e.g., ⑦ in FIG. 2A and FIG. 2B) into the target nucleic acids. These addition events produce first complexes, which contain multiple copies of insertion oligonucleotides inserted into the target nucleic acid fragment. By controlling the reaction conditions, e.g., ratio of capture oligos, beads, target nucleic acid, the distance between inserted can be controlled so that insertion sequences are positioned at approximately regular intervals.

The length of the target nuclei acid fragments, from which first complexes are generated, can vary over a wide range. In some embodiments, the length of the target nuclei acid fragments may be 10kb-100 megabases in size, e.g., 10 kb - 50 kb, 20 kb-100 kb, 20 kb-300 kb,100-200 kb, 100 kb-500 kb, or 300kb-5000kb in size. As noted above, the transposon and transposase or gapping enzymes concentration are controlled such that the addition of insertion oligonucleotides occurs infrequently, leaving intervals between adjacent insertion oligonucleotides. The intervals in the target nucleic acid fragment, each defined by two adjacent insertion oligonucleotides, are referred to as first subfragments (e.g., ⑧ in FIG. 2B). The length of the first subfragments typically ranges 1 kb -20 kb, e.g., 1 kb-10 kb, 1 kb-5 kb, or 3 kb-15 kb. The introduction of insertion oligonucleotides by transposition creates nicks in the target nucleic acid fragments and the transposases remain bound to and connect the first subfragments produced by the insertion events. In some embodiments, the first insertion of sequence occurs in solution, i.e., the insertion oligonucleotides are not attached to any solid support. In other embodiments the insertion oligonucleotide is attached to a solid support, e.g., a micron sized magnetic bead.

### 2. First Capture

The first subfragments produced above can be captured using beads with many copies of first capture oligonucleotides immobilized thereon. See FIG. 2A. Each of the first capture oligonucleotides may comprise a first barcode and a UMI. The first barcode is unique to each bead, i.e., all the first barcodes in the capture oligonucleotides of the same bead have the same sequence. The UMI is a random or semi-random sequence, which differ among the first capture oligonucleotides on the same bead in sequence. The first capture oligonucleotides may also comprise a T7 promoter or a PCR primer binding site. In one approach, the first capture oligonucleotides further comprises a first common sequence ⑤, which hybridizes to a portion of a splint oligonucleotides (④). The splint oligonucleotides includes another portion that can hybridize to the hybridization sequence (⑥) in the insertion oligonucleotide. A DNA ligase is added to ligate the capture oligonucleotides to the insertion oligonucleotides in the first complexes. These steps result in transfer of the first barcode to the first subfragments in the first complexes and produces barcoded first subfragments, each comprising at least one barcode sequence (e.g., one or two first barcode sequences). In another approach, the capture oligonucleotide is hybridized to the insertion oligonucleotides linked to the first subfragments. The insertion oligonucleotides are then extended to produce the barcode sequence.

### 3. Performing first insertion and first capture in one step

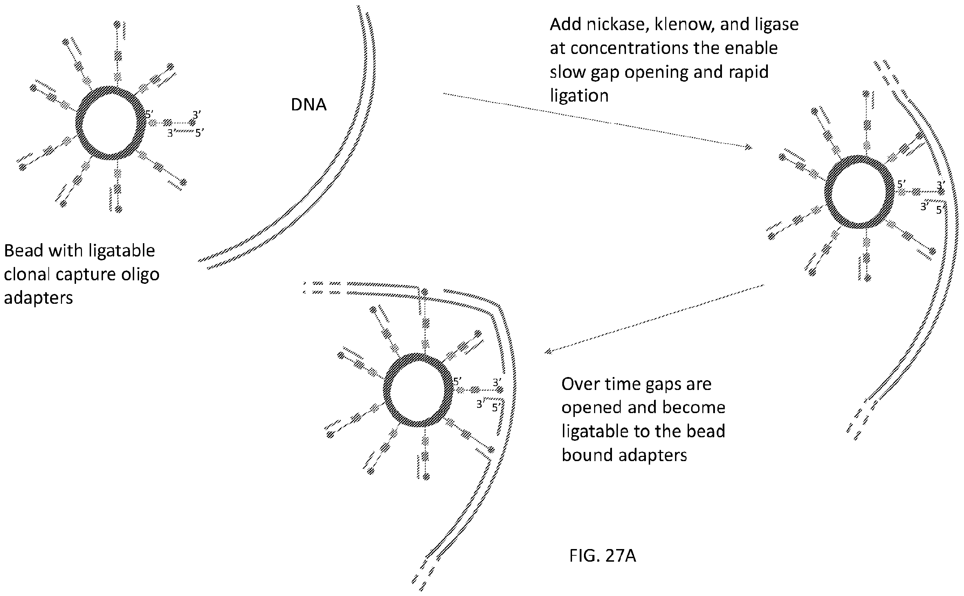

Alternatively, Steps 1 and 2 above can be combined as one step. In another word, the insertion of oligonucleotide into fragments of the target nucleic acid and capture of the first subfragments to beads occur simultaneously. In one approach, the insertion oligonucleotides are hybridized to the capture oligonucleotides that are immobilized on the beads to form a partially double-stranded molecule that is suitable for branch ligation, and introduction of the insertion oligonucleotide (through a branched ligation reaction) occurs on the surface of the beads. See FIG. 27A. In another approach, insertion oligonucleotides are hybridized to the capture oligonucleotides that are immobilized on the beads to form a partially double-stranded molecule that is suitable for branch ligation, and the capture oligonucleotide is ligated to the first subfragments.

Typically, in this approach of performing insertion and first capture in one step, staggered single-stranded breaks are introduced, using gapping enzymes comprising e.g., a nickase and Klenow, into fragments of the target nucleic acid, thereby producing a plurality of first subfragments and all first subfragments generated from a single nucleic acid fragment is collectively called first complex. The method further comprises combining the first complexes and a population of first beads together in a mixture (typically a single mixture, such as a mixture in a single tube). Usually the number of first beads is greater than the number of first complexes in the single mixture so that every first complex is captured by a bead. Each first bead is linked to a first capture oligonucleotide, which is hybridized to a complementary oligonucleotide. In some embodiments, the first capture oligonucleotide comprises (1) a promoter sequence or a primer binding sequence, (2) a first barcode, wherein first capture oligonucleotides immobilized on the same individual bead comprise the same first barcode, and a majority of beads have different first barcodes. In some embodiments, the 3' of the first capture oligonucleotide is linked to the bead. In some embodiments, the 5' of the first capture oligonucleotide is linked to the bead. The method further comprises producing barcoded first subfragments by either (i) ligating the first capture oligonucleotide to the plurality of first subfragments by 3' branch ligation, if the 3' of the first capture oligonucleotide is linked to the bead or (ii) ligating the complementary oligonucleotide to the plurality of first subfragments by 3' branch ligation, if the 5' of the first capture oligonucleotide is linked to the bead and the complementary oligonucleotide to incorporate the first barcode sequence.

In another approach, which combines insertion and capture in one step, transposons comprising capture oligonucleotides as described above are immobilized on the individual beads. Target nucleic acid fragments are combined with the beads, in the presence of a transposase. This allows the capture oligonucleotides being inserted into target nucleic acid fragments. This approach is described PCT publication WO 2014/145820. This approach can be applied to both the first round of stLFR and/or the second stLFR.

### 4. Release

In one approach, the barcoded first subfragments are extended by nick translation using a primer that binds to the PCR primer binding site, resulting in first complexes flanked by the capture oligonucleotides at both ends. Optionally, the extended fragments are released from the beads. The release from beads can be performed by degrading the first beads or by cleaving a chemical linkage between the capture oligonucleotides and the bead. In some cases, the release is effected by removal of an inosine residue from the capture oligonucleotide using EndoV enzyme or the removal of a uracil nucleotide by uracil deglycosylase and EndolV/EndoVIII or other enzymes having similar function. In some cases, the capture oligonucleotide is crosslinked to the bead through one or more disulfide bonds. In such cases, the release can be effected by exposing the beads to a reducing agent (e.g., dithiothreitol (DTT) or tris (2-carboxyethyl) phosphine (TCEP)). Transposases is then removed from the first target DNA fragments by SDS, which results in smaller DNA fragments, the first subfragments. Each about 10kb-20kb.

The barcoded first subfragments in a first complex can also be separated, by denaturing the transposase, if the transposition is used to generate the first subfragments. In the case where the first subfragments are generated using gapping enzymes and that the first subfragments within each first complex are held together due to the presence of overlaps between complementary single-strand fragments. See FIG. 28A ("I"). The first subfragments can be separated by denaturing the first complex.

### 5. Amplification

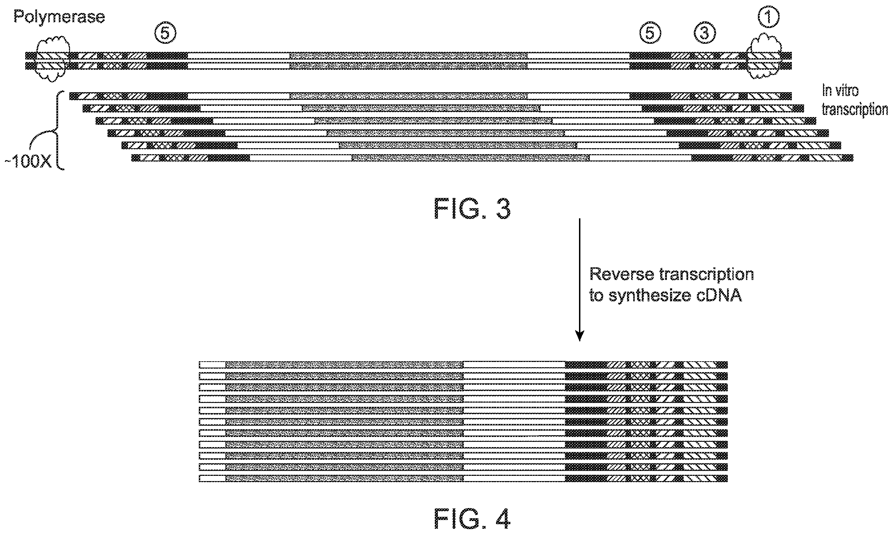

The barcoded first subfragments can be amplified in a variety of ways. In some cases, the first amplification is linear amplification. In some embodiments, where the capture oligonucleotides carry a promoter (e.g., T7 promoter), the linear amplification is performed by in vitro transcription (FIG. 3) coupled with reverse transcription to produce the first strand (FIG. 4). The second strand synthesis can be carried out afterwards to generate a double-stranded DNA molecule (not shown). In some embodiments, the amplification of the barcoded first subfragments comprise (1) performing in vitro transcription to generate RNA transcripts, (2) reversely transcribing the transcripts using a primer annealing to the promoter sequence to generate cDNA strands of the barcoded first subfragments. Optionally, the cDNA strands circularized and are further amplified by rolling circle amplification, as further described below. Double-stranded, barcoded first fragment can be produced using cDNA strands as templates.

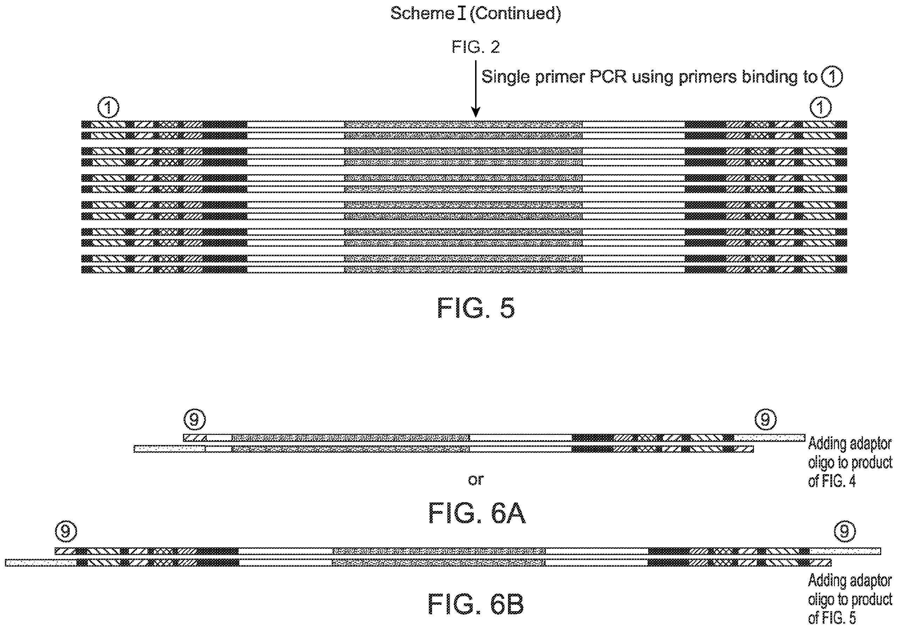

In some embodiments, the amplification is performed using a single primer PCR. In some cases, the PCR primer can be the primer that recognizes the primer binding site in the capture oligonucleotides that flank the first subfragments. Single primer amplification is typically performed for only a few cycles, for example, 8 cycles, to achieve e.g., 100x amplification. See FIG. 5. In some embodiments, the amplifying the barcoded first subfragments is by extending the barcoded first fragments using a primer binding to the primer binding sequence in the first capture oligo; release the extended, barcoded first subfragments from beads, and amplifying the released barcoded first subfragments by single primer amplification by about 80-120 fold, whereby producing amplified, barcoded first subfragments.

### 6. Ligate adaptor oligos

In some cases, after the single primer amplification, an adaptor oligonucleotide (⑨) is ligated to both ends of the amplified, barcoded first subfragments (FIG. 6A or FIG. 6B). In some embodiments, about 3-100x, e.g., 5-50 fold, 2-20 fold, 10-60 fold amplification is performed using any of these methods. The adaptor oligonucleotides is can be a double-stranded or a partially double-stranded DNA molecule.

### 7. Second insertion

A second insertion can be performed using approaches described herein above in relation to the first insertion. In some embodiments, the inserted oligonucleotides used in the second insertion ("second insertion oligonucleotides") has the same as the insertion oligonucleotides used in the first insertion. In some embodiments, the inserted sequence used in the second insertion is different from the sequence used in the first insertion. In some embodiments, the enzymes used in the second insertion are the same as the enzyme used in the first insertion. In some embodiments, the enzymes used in the second insertion are different from the enzyme used in the first insertion. In some embodiments, both the first insertion and the second insertion are performed by transposition. In some embodiments, the first position is performed by transportation while the second insertion is performed using gapping enzymes (e.g., a nickase used with a ligase) as described above. In some embodiments both the first insertion and second insertion are performed by enzymes comprising a nickase and a ligase.

Typically, the insertion frequency of the second insertion is higher than that of the first insertion, such that the fragments between two adjacent second insertion oligonucleotides are shorter than those produced by the first insertion, i.e., the fragment between two adjacent first insertion oligonucleotides. In some embodiments the second subfragments are typically 100-1000 bp in length.

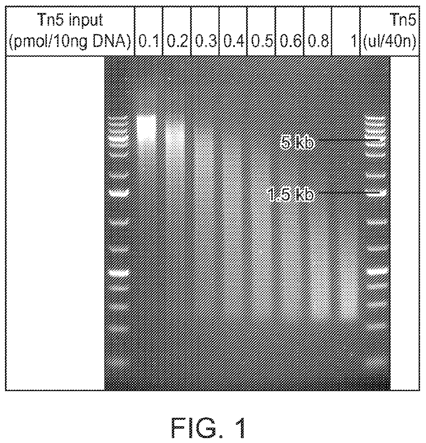

A higher frequency of insertion can be achieved by e.g., using higher concentrations of transposons and/or higher amount of transposases or higher amounts of gapping enzymes, ligase, and adapters. Typically increasing concentrations of insertion enzymes resulted in higher frequency of insertion, which results in smaller DNA fragments between the insertion sites. As shown in FIG. 1, a transposon and Tn5, used at different concentrations in a range from 0.1 pmol/10ng genomic DNA to 1 pmol/10ng genomic DNA, produced differently sized genomic fragments: the higher concentration of Tn5 and the transposon the smaller the fragments between the insertion oligonucleotides.

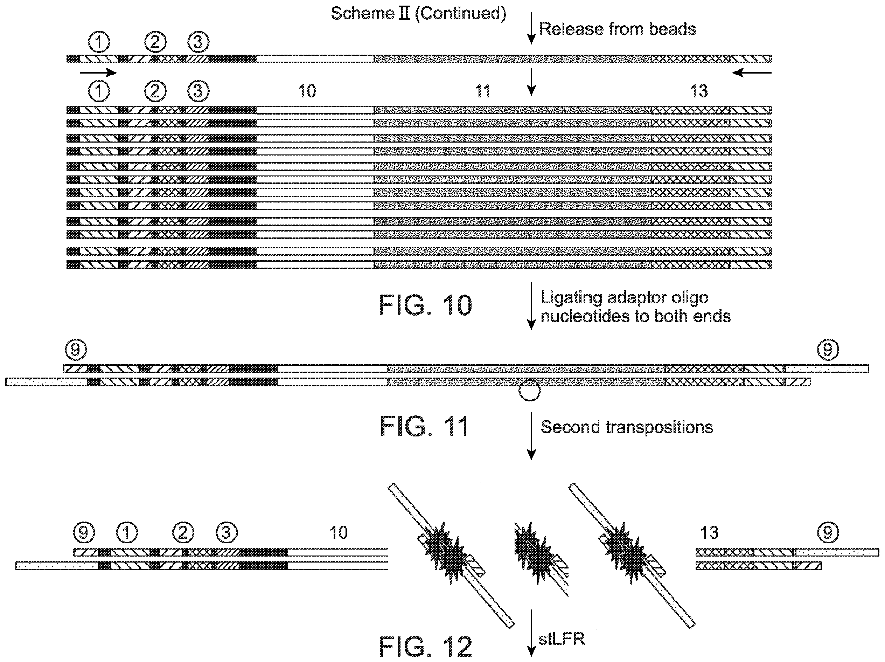

The second insertion introduces second insertion oligonucleotides into each of at least some of the amplified, barcoded first subfragments, thereby producing a plurality of second subfragments, collectively called the "second complex". Each second complexes contains multiple copies of the second insertion oligonucleotides. See FIG. 12. In preferred embodiments, the second insertion oligonucleotide has the same sequence as the sequence of the adaptor oligonucleotides (⑨) in Figures 6A and 6B). Similar to the first insertion, the second insertion creates second complexes, each of at least some of the second complexes comprises a plurality of second subfragments. Each of the second subfragments is located between one second insertion oligonucleotide and its flanking second insertion oligonucleotide. In the case of using transposons the transposases, these second subfragments are interconnected through transposases that remain bound to the ends of the second subfragments. In some embodiments, the second insertion occurs in solution, i.e., the inserted sequences are not attached to any solid support. The length of the second complex typically ranges 1 kb - 20 kb, e.g., 1 kb-10 kb, 1 kb-5 kb, or 3 kb-15 kb.

### 8. Second Capture

A second round of capture is performed in which the second complexes are captured by second beads, in a way similar to the first round of capture. Each second bead has many copies of second capture oligonucleotides immobilized thereon. Each second capture oligonucleotides comprises a second barcode that is unique to each second bead, i.e., all the second barcodes in each second bead are the same. The second capture oligonucleotides also comprise a primer binding site and a second common sequence. The second common sequence hybridizes to a portion of a second splint oligo. The second splint oligonucleotides includes another portion that can hybridize to the hybridization sequence in the second insertion oligonucleotide. A ligase can be added to ligate the second capture oligonucleotides of individual beads to the second insertion oligonucleotides in the second complexes. In another approach, the capture oligonucleotide is hybridized to the second insertion oligonucleotides linked to the second subfragments. The second insertion oligonucleotides are then extended to produce the second barcode sequence. As one of skill in the art would appreciate, any of the approaches described for introducing first barcodes to first subfragments can be used to introduce the second barcodes to second subfragments. These steps result in barcoded second subfragments, each comprising at least one second barcode sequence (e.g., one or two second barcode sequences).

### 9. Perform second insertion and second capture in one step

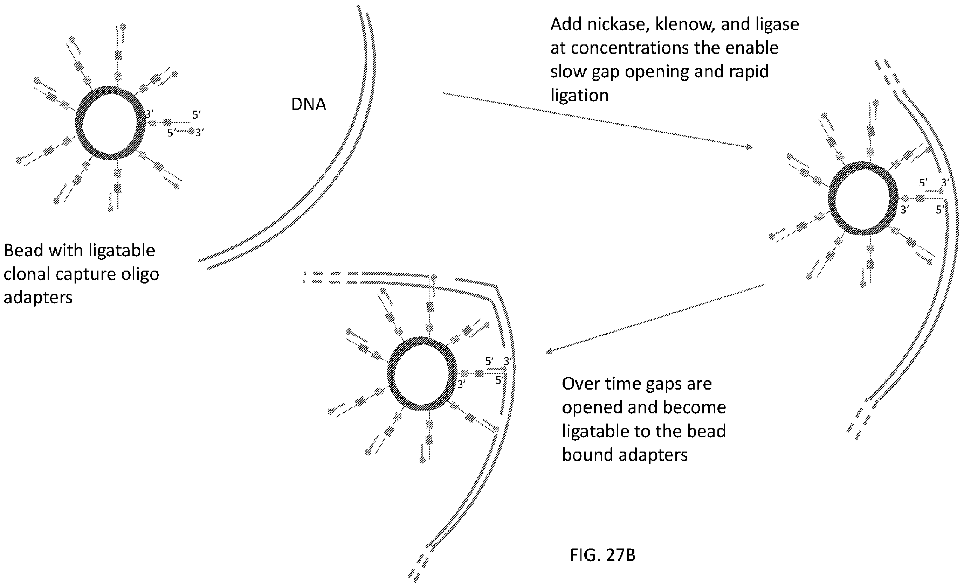

In an approach similar to performing first insertion and first capture in one step, second insertion and second capture, as Steps 7 and 8 above, can also be combined in such a way that second insertion oligonucleotides are already immobilized on the beads, and addition of the second insertion oligonucleotide and generation of second complexes occur on the surface of the beads. See FIG. 27A and 27B. In this embodiment, the method comprises introducing staggering single-stranded breaks into fragments of the target nucleic acid, thereby producing second complexes. Each second complex comprises a plurality of second subfragments and each second subfragment located between two adjacent single-stranded breaks. The method further comprises combining in a single mixture the second complexes with a population of second beads. The number of second beads is greater than the number of second complexes in the single mixture so that every first complex is captured by a bead. Each second bead comprises multiple copies of double-stranded or partially double-stranded capture oligo adaptors immobilized thereon. Each adaptor comprises one strand that is a second capture oligonucleotide that is linked to the bead and one strand that is complementary to the second capture oligonucleotide. In some embodiments, the second capture oligonucleotide strand comprises (1) a promoter sequence or a primer binding sequence, (2) a second barcode, wherein second capture oligonucleotides immobilized on the same individual bead comprise the same second barcode, and a majority of beads have different second barcodes. The method further comprises producing barcoded second subfragments by either (i) ligating the second capture oligonucleotide adaptor to the plurality of second subfragments by 3' branch ligation, or (ii) ligating the strand that is complementary to the second capture oligonucleotide to the plurality of second subfragments by 3' branch ligation, and the extending the complementary strand to incorporate the second barcode sequence.

### 10. Branch ligation

Optionally, the second capture oligonucleotide comprises a first PCR primer annealing site. Optionally, a 3' branch ligation adaptor oligonucleotide is ligated to the second subfragments at the nicks, wherein the 3' branch ligation adaptor comprises a second primer annealing site. Branch ligation is described in detail in Wang et al., BioRxiv, June 29, 2018, doi:https//doi.org/10.1101/357863; and PCT publication no. WO 2019/217452.

### 11. Prepare second subfragments for sequencing

Individual second subfragments are prepared by either removing the enzymes (e.g., using SDS) that are bound to the ends of the second fragments or by denaturing the second complex. The individual second subfragments are then amplified using two primers, one annealing to the first PCR primer annealing site and the other annealing to the second PCR primer annealing site. The amplified fragments can be sequenced as described below

The combination of the two barcodes and the UMI allows for construction of intermediate fragments, i.e., the first subfragments, by combining sequence reads having the same second barcode. The sequences from the intermediate fragments are then used to construct the complexes target nucleic acid fragment by combining sequences of the intermediate fragments that have the same first barcode. This allows constructing sequence of long target nucleic acids.

### 12. Rolling circle amplification

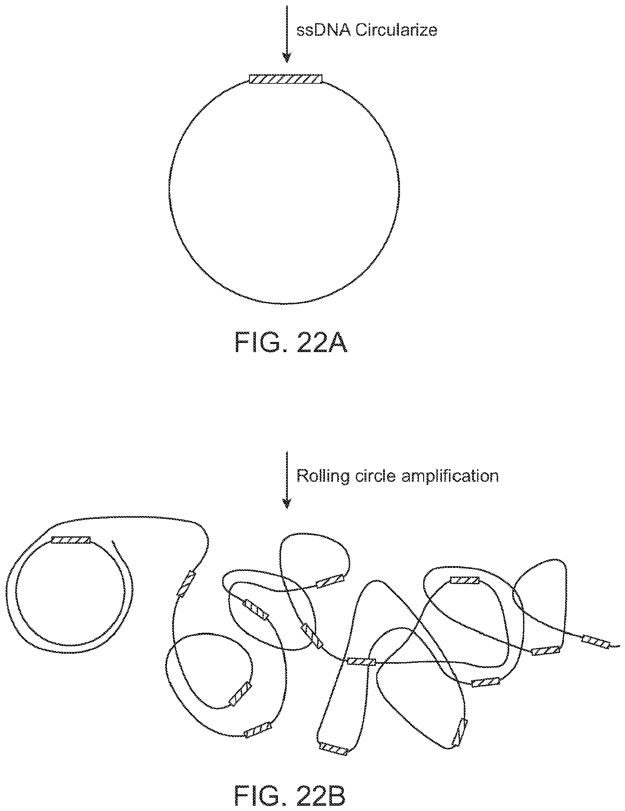

As described in step 4, above, in some embodiments, the amplification can be performed using rolling circle amplification ("RCR") to enable PCR-free long fragment read analysis. See FIG. 22A and 22B. In some embodiments, RCR is desirable as it has the advantages of amplifying long nucleic acid fragments, including full-length genome, rather than small fragments. RCR typically has higher fidelity than PCR, in which errors generated in earlier cycle of PCR are carried over to the later cycles of amplification. In addition, RCR requires only one primer while PCR typically requires two primers to carry out the amplification. Also, copies of the original sub-fragment are connected together as a single concatemer allowing capture to a bead as a single molecule with many copies of the same sub-fragment.

Rolling circle amplification requires a circular template. In some embodiments, the barcoded first subfragments, as described in step 4, above, are denatured into single-stranded nucleic acid molecules. A splint oligo is then added and the single-stranded nucleic acids are then circularized in the presence of a ligase (e.g., T4 or Taq ligase).

The DNA polymerase used for RCR can be any DNA polymerase that has strand-displacement activity, e.g., Phi29, Bst DNA polymerase, Klenow fragment of DNA polymerase I, and Deep-VentR NDA polymerase (NEB#MO258). These DNA polymerases are known to have different strength of strand-displacement activity. It is within one of ordinary skill in the art to select one or more suitable DNA polymerase used for the invention.

In some embodiments, the barcoded first subfragments are amplified by linear amplification and the amplified products are the circularized in a similar manner to serve as the circular template for RCR. In some embodiments, the linear amplification the precedes the RCR is a 2-10x, e.g., 2-3x, 2-5x, or 3-10x amplification. This amplification is desired because it provides redundancy to cover almost all barcoded fragments with at least one tagmented concatemer bound to the second barcoding beads in spite of substantial DNA losses during processes of circularization concatemer generation, and capture by barcoded beads. This combination, i.e., linear amplification and RCR, allows for more than 90% of the bases of the initial long fragments to be represented by at least one strand, if the initial barcoding efficiency is 70-80%. For many applications more than 70% or more than 80% would be sufficient. The more coverage the fewer number of cells. Thus the method can be used to analyze a number of cells that is less than 50, less than 30, less than 20, less than 15, less than 10, or less than 5 human or other cells.

### 13. PCR free long fragment analysis

In some embodiments, the preparation of long nucleic acid fragment for sequencing analysis are PCR-free, i.e., the amplification is by RCR only. See FIG. 22A and 22B. The number of cells used for the analysis can vary widely, e.g., 5-50 cells, 3-20 cells or 10-100 cells or more. Equivalent amounts of DNA can be used from large pools of genomic DNA isolated from a much larger number of cells, e.g. thousands to billions). Cells can be dispensed into a single tube or single cells can be added to individual wells of a microwell plate for single cell analysis. Cells are lysed and DNA is released from the cells. To achieve this a lysis and protease treatment can be performed as described in C. Chen et al., Single-cell whole-genome analyses by Linear Amplification via Transposon Insertion (LIANTI). Science 356, 189-194 (2017)).

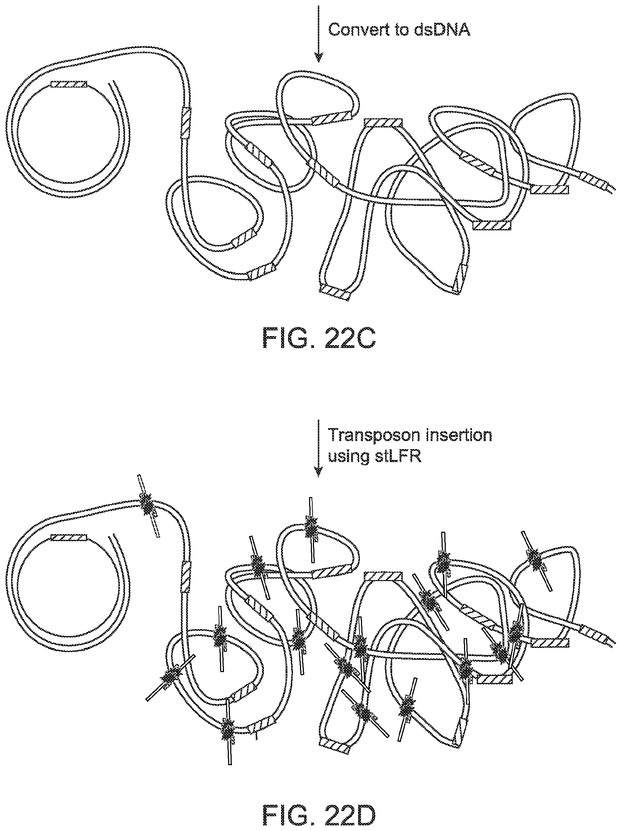

Genomic DNA from the cells can be amplified, and these amplified products, if they are single-stranded (e.g., produced by rolling circle amplification), are first converted to double-stranded or partially double-stranded DNA molecules. These DNA molecules, which are analogous to the target nucleic acid fragments as described above, can be subjected to the first insertion, first capture, etc. in the manner similar to what is described above. As described above, in one approach, transposons coupled with transposases are incubated with amplified products so that insertion oligonucleotides are inserted into the amplified products. The interval between adjacent insertion oligonucleotides introduced by the transposons has a length ranging from 1kb to 50 kb, e.g. from 1kb to 3kb, from 1kb to 5kb, from 2kb to 5kb, from 3kb to 6kb, from 3kb to 10kb, from 5kb to 20kb, or from 10kb to 50kb. If the amplified products are single-stranded DNA, they are converted into double-stranded DNA before transposition. See FIG. 22C and 22D. Alternatively DNA can be prepared for co-barcoding by using techniques such as nicking/gaping before or during interaction of long DNA with barcoded beads. The result is that the interval between adjacent insertion oligonucleotides has a length that ranges from 300,000 bp to 3,000,000 bp.

For single cell analysis, transposons with unique barcodes are dispensed into each well (or other compartment), each well containing no more than one cell. In this way each DNA from individual cells is tagged with a specific barcoded transposon for that cell. As described above, in some embodiments, transposon-inserted DNA can be add incubated with beads having average diameter ranging from 1-50 um. Each bead carries between 1×10³-1×10⁹ capture oligonucleotides which share an identical barcode as described previously in, e.g., US 2014/0323316. In some embodiments, 1-100×10⁶. beads could be used. After target nucleic acid fragments are captured through hybridization to the hybridization sequence of the transposon and the capture oligonucleotide on beads, ligation is performed so that the barcodes are inserted into the target nucleic acid fragments. At this point the transposase enzyme is disrupted and the subfragments created by transposon insertion are separated from each other, but still linked to the beads. Typically about a 50-80% yield can be expected at this step, which means that 2.5-35 cells equivalent DNA would remain and 75-91% of the original long fragment is represented with barcoded DNA. An extension step can be performed to copy part of the transposon sequence on the 3' side of the captured fragments. This sequence will be used as a hybridization sequence for a splint oligo if single strand circularization is performed, otherwise it can be used as a primer for extension. In addition, the part of the transposon sequence can also be used to ligate an adapter to the 3' recessed ends created by transposon insertion to create a sequence for primer extension and splint oligo hybridization. At this point linear amplification can be performed on the beads through successive rounds of denaturation, hybridization of primers and extension or PCR can be performed if ligation of an adapter to the 3' end of each fragment to add an additional primer site was used.

In addition, initial barcoded fragments (aka, barcoded first subfragments) can be prepared for amplification (e.g., rolling circle amplification) by first releasing the fragments from the beads through, e.g., removal of an inosine residue from the capture oligonucleotide molecule using EndoV enzyme. Double-stranded or partially double-stranded are produced using the barcoded first subfragments as templates. Next the dsDNA fragments are denatured, splint oligo is added, and ssDNA circularization is performed using T4 or Taq ligase. See, FIG. 22A. DNA generated by linear amplification can also be used for circularization.

If using a thermostable ligase such as Taq ligase, cycles of denaturation and ligation can be performed to increase yield (e.g., to at least about 70%). We expect about a 70% yield at this step leaving 1.75-24.5 cells of DNA remaining. However, both strands of the DNA can be captured suggesting that up to 75% (0.7x0.7=49% of fragments circularizes; 0.51×0.51=~25% of segments would not be represented by either strand) of each fragment will be covered by at least one strand of the DNA.

Alternatively, a 2-3 fold or 2-5 fold or 3-10 fold linear amplification can be performed before circularization, which makes 10-100x concatamers. This provides redundancy to cover almost all barcoded target nucleic acid fragments with at least one tagmented concatamer bound to the second barcoding beads, despite of substantial DNA losses during the steps of circularization, concatamer generation, and binding it to barcoded beads.

This approach allows for >90% of bases of the initial long fragments to be represented by at least one strand if initial barcoding efficiency is 70-80%. For many applications representation higher than 70% or >80% is adequate. The more coverage the fewer number of cells is needed for sequence determination. Sequencing can be carried out using the methods described herein starting with fewer than 50 cells, or <30, <20, <15, <10, <5 cells (e.g., human cells).

About 3-100 fold (preferably 10 fold) amplification is performed using any of these amplification methods described above. For rolling circle amplification and linear amplification, ssDNA fragments can be converted into dsDNA by annealing primers complementary to adapter sequences and performing extension with a DNA polymerase. In some embodiment, the DNA polymerase has nick-translation capability. Ligases may also be used to seal the nicks. With all molecules converted to dsDNA, transposons can be inserted again at a frequency of 0.1-1.5 kb (i.e., the length of the interval between adjacent insertion oligonucleotides ranges from 0.1 kb to 1.5 kb) preferably 0.3kb-1.5kb. These transposon inserted fragments are captured to barcoded beads as described in above.

### 14. Fractioning circles by size

In some embodiments, it is advantageous to separate the circles (e.g., the circles formed by circularizing the first subfragments, see above) by size before rolling circle amplification (RCA or RCR) to achieve similar read coverage for variable fragments resulting from the first barcoding. With sufficient amplification, size selection of the barcoded fragments before preparing for sequencing may be used to obtain fragments from e.g. 300-500 base or 400-600 base to achieve more efficient sequencing. Also preparing arrays (either by DNBs or clusters) can be more efficient with sufficiency of DNA. For 15 cells and covering 6×10⁹ bases per cell in 3 kb fragments, there are 3×10⁶ fragments each converted into ^{~}100 kb concatamers. Thus, 30×10⁶-100×10⁶ or more barcoded beads are needed.

In some embodiments, size selection is achieved by fractioning circles. In some embodiments, fractioning circles is performed by capturing circles on solid supports (e.g., beads) by hybridization, which is followed by controlled primer extension with a strand-displacement polymerase to displace circles from the beads. Capturing by hybridization may be carried out by, e.g., mixing the circle with a solid support having an oligo immobilized thereon, where the oligo can hybridize to a sequence in the circle. Preferably, the sequence in the circle that hybridizes to the immobilized oligo does not overlap with the sequence in the circle the extension primer binds. The extension length may be controlled by selecting a polymerase(s) with a suitable polymerization rate or other properties, and by a variety of reaction parameters, including but not limited to, reaction temperature, DNA polymerase concentration, primer concentration, and dNTP concentration. Optimal conditions may be determined empirically. The DNA polymerase used for circle fractioning can be any DNA polymerase that has strand-displacement activity as described above. In some embodiments, the DNA polymerase is Phi29. In some embodiments, the reaction temperature is between 2°C and 30°C, e.g., between 2°C and 20°C, or between 10°C and 25°C. In some embodiments, a limited amount of dNTPs may be used that allow only pre-defined extension length, e.g. 300 bases. Primer extension reaction would practically stop after reaching ^{~}300b in this case because of using almost all dNTPs provided.

The displaced circles are released into the supernatant of the reaction mixture. Under the same conditions for primer extension, including, e.g., polymerase speed, polymerase concentration, dNTP concentration, a longer extension reaction is needed to displace larger circles from the solid supports than smaller circles. By collecting supernatants at predetermined time intervals after the initiation of polymerization, circles with the desired sizes can be collected. In some embodiments, the supernatants containing the circles released from the solid supports are collected at 2, 3, 4, or 5 different timepoints after initiation of the primer extension.

The circles with desired sizes are then amplified by rolling circle amplification to produce concatemers with similar number of copies of target DNA. Typically, the larger the circles, the longer the time of rolling circle amplification is used.

To ensure effective circle fractioning, it is desirable to ensure that the circles start rolling at approximately the same time. Non-limiting exemplary methods of ensuring approximately simultaneous rolling of the circles include using a high concentration of polymerase (e.g., at least 10 fold excess of polymerase relative to circles, for example, in the range of 10 to 1 million fold more polymerase than circles) for nucleotide polymerization; hybridizing extension primers to the circles to form hybridization complexes before capturing the hybridization complexes on the solid supports, incubating DNA polymerase with the circles (e.g., in the presence of EDTA) before capturing the circles on the solid supports.

Circle fractioning disclosed herein can also be used in many other applications, such as size selection for regular sequencing libraries (e.g. to get sizes from about 400-600 bases, or 500-1000b or 200-400b or 300-600b) or equalization of copy number for DNBs used for sequencing (to get similar sequencing signal/intensities) or other purposes.

### 15. Sequence barcode for RCR products

First in converting ssDNA concatemers to dsDNA ligase can be used in addition to proper polymerase to seal nicks directly using phosphorylated primers or by nick-translation-ligation. Second, over 2X sequencing coverage per adapter (with first barcode) + DNA fragment unit is needed (preferably 3X or more) to assure that in >90% or >95% cases barcode is sequenced. 3X coverage also allows to assemble sequence per each ^{~}3 kb fragment ("synthetic ^{~}3kb"; mostly as one or 2-5 contigs is some cases). This would help *de novo* assembly. In addition, being able to separate reads from ^{~}3 kb fragments of the original long DNA molecule can be very powerful in de novo assembly and especially in regions of repeated sequence. Even with less read coverage per ^{~}3 kb fragments, localization of reads in 3 kb regions is very informative. This is similar to the previously described strategy of intermediate barcoding with linked barcoded transposons.

Using 3X coverage per unit fragment and 15 cells × 2 chromosomes (assuming on average 50% of DNA in each strand is barcoded in the second step which is equivalent to having only one strand), the total coverage is ^{~}90X or 300 Gb per human genome. If making concatamers is >90% efficient, capture of concatamers on beads is 90% efficient, and sequencing coverage is >90%, then 0.50 DNA in circles × 0.73 of bases in circles sequenced = 37% of each strand or ^{~}60% in at least one strand of an original long fragment is covered.

### 16. Population sequencing of long DNA targets

One or more target DNA fragments (e.g. 5-50kb, or 10-50kb or 10-300kb or 30-300kb) can be isolated or enriched from the genomic DNA of a population of hundreds, thousands or millions of cells or organisms like bacteria or fungus or others. The enrichment can be performed by target specific long-range PCR or by hybrid capture or by other methods. The prepared dsDNA would go through the here-described double stLFR process for cost effective single long DNA molecule sequencing. >80% or >90% or >95% of all bases would be sequenced in each of thousands to millions of 10kb+ DNA molecules.

The applications can include deep sequencing of whole gene or gene clusters for detecting cancer or other somatic mutations or variants (such as drug/antibiotic resistance) in pathogen populations or diversity of target genes in microbial populations, e.g. gut microbiome.

### 17. Novel genomics libraries

Using the methods disclosed herein, a genomic library comprising >50%, 70%, 80%, 90%, 95% of bases of each of a large plurality of long genomic DNA fragments (e.g. 10-50kb, 10-300kb, 20-300kb, 20-100kb) can be obtained. These long genomic DNA fragments are represented in the barcoded templates in the form of plurality of subsets of templates belonging to shorter subfragments (e.g. ^{~}1-10kb, ^{~}1-5kb, ^{~}3-15kb) of long fragments. The mathematical representation of these novel libraries are shown below. Each of the long fragment has a unique barcode (e.g., Long fragment 1 has barcode 1.0) that is different from the barcodes of other long fragments (e.g., Long fragment 2 has barcode 2.0). Each subset (e.g., "subfragment 1.1 subset") belongs to a long fragment (e.g., "Long fragment 1") that has a specific barcode (e.g., "barcode 1.0") that is different from other subsets (e.g., "subfragment 1.2 subset") belonging to the same long fragment, and at least one template from each such subset also has a barcode specific for the long DNA (e.g., at least one template of subfragment 1.1 subset has both barcode 1.1 and barcode 1.0).

### 18. Mathematical representation of novel libraries:

Long fragment 1 co-barcoded with barcode 1.0
Subfragment 1.1 subset: multiple templates with barcode 1.1 + at least one template with barcode 1.1 and barcode 1.0.
Subfragment 1.2 subset: multiple templates with barcode 1.2 + at least one template with barcode 1.2 and barcode 1.0.
Subfragment 1.n subset: multiple templates with barcode 1.n + at least one template with barcode 1.n and barcode 1.0; n is about 2 to 1000, or 2-100, or 3 to 10, or 3-30, 3-300, or 10-30, 10-100.

Long fragment 2 co-barcoded with barcode 2.0
Subfragment 2.1 subset: multiple templates with barcode 2.1 + at least one template with barcode 2.1 and barcode 2.0.
Subfragment 2.2 subset: multiple templates with barcode 2.2 + at least one template with barcode 2.2 and barcode 2.0.
Subfragment 2.n subset: multiple templates with barcode 2.n + at least one template with barcode 2.n and barcode 2.0; n is about 2 to 1000, or 2-100, or 3 to 10, or 3-30, 3-300, or 10-30, 10-100.
Long fragment F co-barcoded with barcode f.0Subfragment f.1 subset: multiple templates with barcode f.1 + at least one template with barcode f.1 and barcode f.0.
Subfragment f.2 subset: multiple templates with barcode f.2 + at least one template with barcode f.2 and barcode f.0.
Subfragment f.n subset: multiple templates with barcode f.n + at least one template with barcode f.n and barcode f.0; n is about 2 to 1000, or 2-100, or 3 to 10, or 3-30, 3-300, or 10-30, 10-100.; F is 1k+,10k+, 100k+, 1M+, 10m+.

Such libraries can be for plurality of cells as a mixture or a single cell. In some embodiments, the such libraries are prepared from a plurality of cells where each cell has a designated barcoded in addition to the barcode of the long DNA fragment.

### Scheme II. Single cell transcriptome method

This scheme described below can be used to analyze transcripts in cells. Single cells are obtained from the starting population of cells. In some cases, the cells are diluted in a microwell plate. By using a plate in which the number of wells greatly outnumbers the cells, a very high proportion of the wells contain one cell or none no cells. In this scenario, the following method can be used to analyze transcripts from a single cell.

### 1. mRNA Capture and reverse transcription

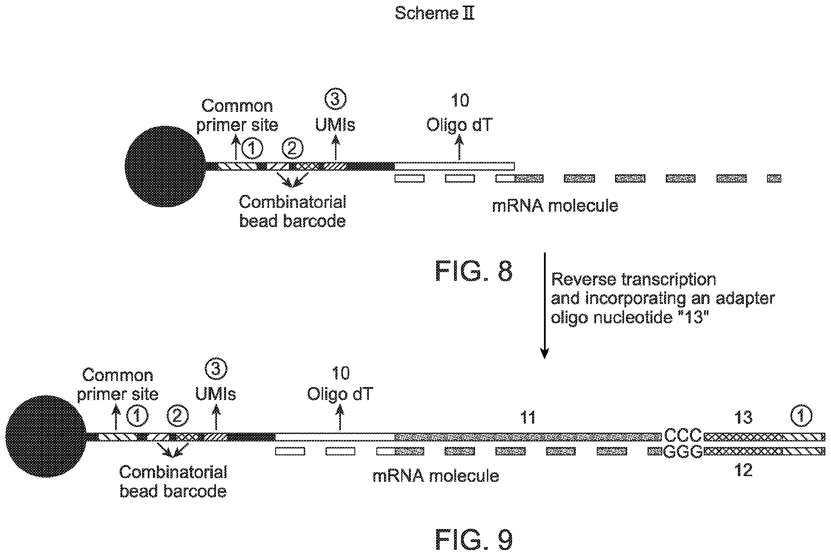

Cells are lysed and mRNA is extracted. The mRNA is combined, in a single mixture, with a population of first beads, each having multiple first capture oligonucleotides immobilized thereon. Each first capture oligonucleotides comprises a unique first barcode sequence, a UMI, a common primer site, and oligo-dT, which is used to capture polyA tail of the mRNA molecules. See FIG. 8.

The captured mRNA molecules are then reverse transcribed to produce cDNA each of at least some of the cDNAs are linked to the first capture oligonucleotides on the first beads to form a cDNA/mRNA hybrid molecule. FIG. 9. In some embodiments, the cDNA/mRNA hybrid molecule produced as above is then ligated to an adaptor comprising a GGG tri-nucleotide, and which are complementary to the CCC sequences added to the 3' end of the cDNA by the reverse transcriptase. See FIG. 9. The adaptor may also comprise a PCR primer binding sequence, which may be the same as the common primer binding sequence in the capture oligonucleotide. The CCC tri-nucleotides can be added to the 3' end of cDNA when reverse transcriptase reaches the 5' end of the RNA template under conditions such as high concentrations of magnesium and/or manganese ions. Methods for inducing the addition of the CCC tri-oligonucleotides are well known in the art, for example, Schmidt et al. Nucleic Acids. Res. 27(21):e31 (1999); Pinto et al., Anal. Biochem. 397(2): 227-232 (2010).

### 2. Release and amplification

Optionally the cDNAs are released from the first beads. In some embodiments, the first fragments are amplified with a common primer that recognizes the common primer binding sequence on the first capture oligo and also the primer binding sequence on the adaptor, both integrated into the synthesized cDNA. See FIG. 10. The advantage of using single primer amplification is that the single primer amplification more likely results in hairpin formation for shorter amplification product. These hairpin structures will not be further amplified do not participate in downstream processing steps. Thus using single primer PCR in this step can increase the number of longer amplification product.

Optionally the molecule is circularized and amplified by rolling circle amplification.

### 3. Ligate adaptor oligonucleotides to the amplified double-stranded DNA

The amplified double-stranded DNAs are ligated with adaptor oligonucleotides at both ends. Each adaptor is a partially double-stranded DNA. See FIG. 11.

### 4. Perform second transpositions and single tube LFR as in Scheme I Steps 6-8

The double-stranded DNA with adaptor oligonucleotides at the ends are transposed with second insertion oligonucleotides, followed by incubation with second binds as described in Scheme I, steps 6-8.

The combination of the two barcodes, one from step 1, and another from step 4, and UMI allows for complete construction of all full transcripts and their assign individual cells. For example, when mRNAs extracted from a single cell is incubated with first beads, each of the first barcode corresponds to an individual cell and each of the UMI corresponds to an individual transcript in that individual cell.

### Scheme III. Long amplicon assembly method

The method of Scheme III can be used to analyze the full length of any target region that could be of interest, such as 16s or 18s regions rRNA genes. Some of the embodiments are illustrated in Figures 13-19. The assay can be multiplexed to analyze multiple target regions in a single compartment.

### Scheme IIIa.

### 1. Target specific primer amplification

Each of the one or more target regions is amplified for a limited amount of cycles, e.g., two cycles, with a forward primer, which is composited with special bases that can be degraded by an enzyme, and a reverse primer. The special base is one that does not appear in native DNA. The enzyme can digest the primer through recognition of the special base. In one example, the special base is a Uracil. The forward primer additionally comprises a first common sequence at the 5' and a UMI on 5' end. Each of the forward and reverse primers comprise a target specific sequence ("target specific sequence 1" and "target specific sequence 2" in Figure 13), which allows them to hybridize and amplify target DNA region specifically.

### 2. Remove excessive UMI-containing forward primer

The reaction mixture from step 1 is then treated with the enzyme, which recognizes the special base in the forward primer and degrades it. FIG. 13. In the case where the special base is uracil, USER can be used to digest the excessive forward primer. Because the forward primer contains UMI and target specific sequences, removing the excessive primer can keep UMI from becoming scrambled and allows for multiplexing.

### 3. Further Amplification

DNAs containing target regions from step 2 are amplified using a primer that hybridizes to the first common sequence and the reverse primer to produce the first complexes, double-stranded DNA molecules comprising the first common sequence and target UMI to produce further amplified product. FIG. 14.

### 4. Ligating an adaptor oligonucleotides to the further amplified product

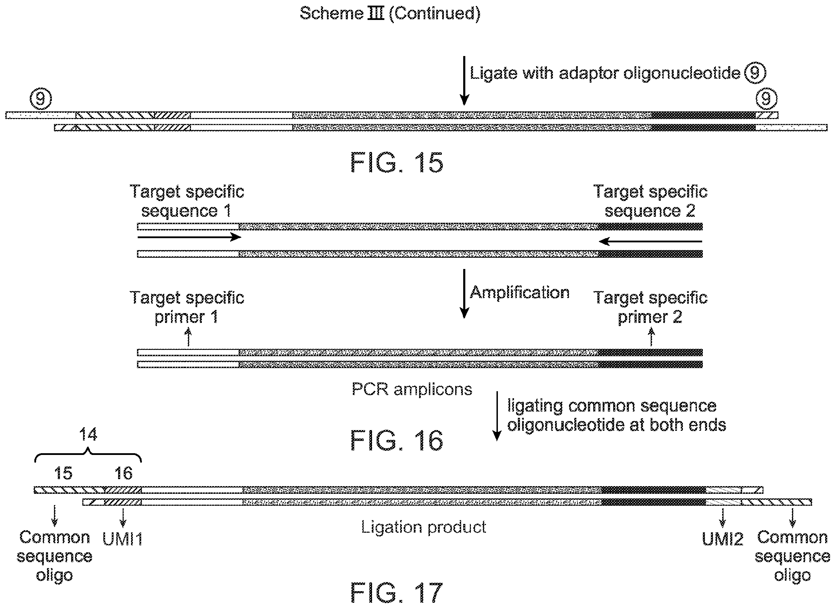

Adaptor oligonucleotides are ligated to the ends of the further amplified product. FIG. 15.

### 5. Transposition and single tube LFR as in Scheme I steps 6-8

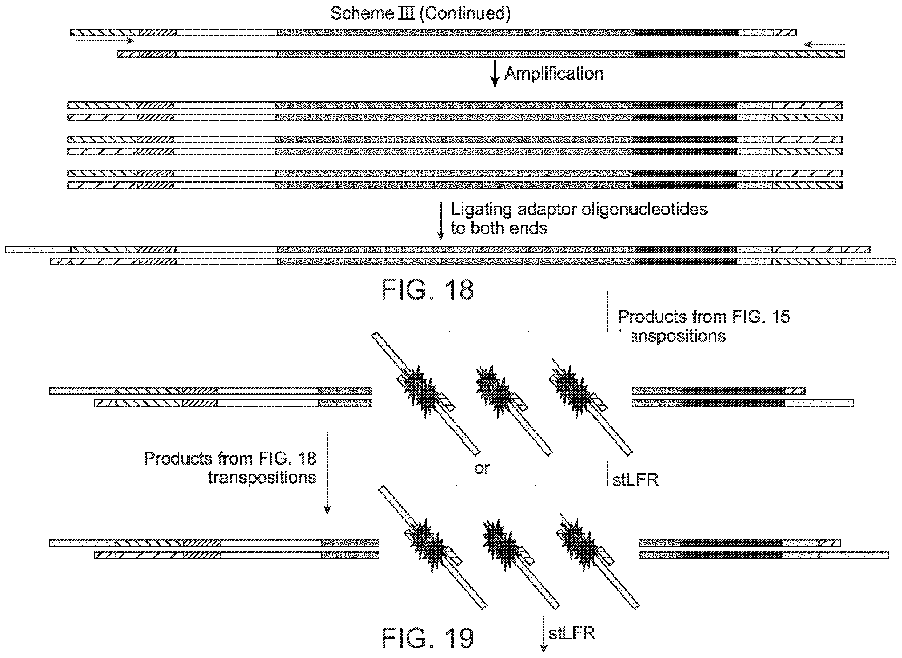

Insertion oligonucleotides, having the same sequence as the adaptor oligo, are introduced into the first complexes through transposition, in a fashion that is similar to Scheme 1, step 6, and StLFR are performed as described in Scheme I, steps 7-8. FIG. 19.

### Scheme IIIb.

### 1. Target specific primer amplification

Each of the one or more target DNA regions is amplified using target specific primer pairs for limited amount of cycles, e.g., two cycles. FIG. 16.

### 2. Adding UMIs and common sequences

Two common sequence oligonucleotides, a first and a second common sequence oligonucleotides, are ligated to the amplified double-stranded DNA comprising the target region, one at either end. FIG. 17. Each of the two common sequence oligonucleotides comprises a common sequence. In some embodiments, the two oligonucleotides share the same common sequence. One or both of the common sequence oligonucleotides may comprise a UMI at 3'. In some embodiments, both oligonucleotides comprise UMIs and the UMIs are different between the two common sequence oligonucleotides. The ligation product is then amplified using primer pairs (forward and reverse primers) annealing to the first and second common sequence oligonucleotides, which are ligated to target DNA regions to produce first complexes. In some embodiments, both the forward and reverse primers have a sequence that is complementary to the common sequence. FIG. 18. In some embodiments, the common sequence oligo is a partially double-stranded oligonucleotide, with the UMI located in the double-stranded region and the common sequence is located in the single-stranded region of the common sequence oligonucleotide. In some embodiments, the common sequence oligo is a partially double-stranded oligonucleotide, with both the UMI and the common sequence located in the single-stranded region.

### 3. Ligating adaptor oligonucleotides

Adaptor oligonucleotides are ligated to both ends of the amplified target regions which are flanked by the first and second oligonucleotides. FIG. 18.

### 4. Transposition followed by single tube LFR as in Scheme I steps 6-8

Insertion oligonucleotides, having the same sequence as the adaptor oligo, are introduced into the first complexes through transposition, similar to Scheme 1, step 6, and StLFR are performed as described in Scheme I, steps 7-8. FIG. 19.

The combination of the two barcodes and the UMI allows for complete construction of the full length sequence of the target region.

### Scheme IV.

In some embodiments, each of the long, target nucleic acid molecule (e.g., about 25 kb to 100 kb) is ligated to an adaptor and a unique molecular identifier (UMI). The UMIs is at a length that is sufficient to distinguish the individual target nucleic acids from one another. The larger the number of target nucleic acid molecules, the longer the UMI needs to be; for example, for 10 million molecules, the UMIs are typically of 11-20, e.g., 11-18, e.g., 12-15 bases or longer. In some cases, the UMIs are prepared in multiple copies before incorporating into the adaptor in order to increase chances of having at least one sequence read comprising a UMI. Thus, in some embodiments, the each of the long, target nucleic acid molecule is ligated to one or more copies of a UMI.

In some embodiments, the target nucleic acid molecule with adaptor and UMI inserted are denatured to form single-stranded nucleic acid molecule, and then circularized. In some embodiments, the double-stranded target nucleic acid molecule with adaptor and UMI inserted are circularized and then denatured to form circularized single-stranded nucleic acid, provided that a nick exists in one strand of the molecule. RCR is performed on the circularized single-stranded nucleic acid molecule to produce a DNA nanoball ("DNB") as described above. The RCR reaction time may vary, but typically the reaction time is of a length such that greater than 5x, e.g., greater than 10x, greater than 20x copies of the target nucleic acid can be produced. For example, for a target nucleic acid molecule that has a length of 2kb-100kb, the reaction time is typically about 10-400 minutes, which produces a DNB that has a length of about 50kb to 1Mb. Thus, in some embodiments, the RCR reaction time is about 10-400 minutes. The DNBs are then converted to double-stranded DNA by annealing primers complementary to the adapter sequence and perform extension with a DNA polymerase. In some embodiments, each DNB is fragmented into multiple fragments, e.g., 3-10 fragments. Fragmentation of the DNB can occur spontaneously, e.g., due to the length of the DNB, or can be achieved by applying force. Transposons are inserted into the double-stranded DNA using a transposase and the transposed DNA are captured to beads, as described in Scheme I, steps 1 and 2. Transposase associated with the double-stranded DNA can then be removed, resulting in detached DNA fragments.

The DNA fragments are then sequenced to produce sequence reads containing the UMI sequence. The presence of the UMI sequence allows association of sequences of multiple DNBs having the same UMI, even if they are captured on different beads. This can advantageously produce close to 1X sequence coverage of the original long target nucleic acid.

### IV. Components of the methods

### 1. Samples

Samples containing target nucleic acids can be obtained from any suitable source. For example, the sample can be obtained or provided from any organism of interest. Such organisms include, for example, plants; animals (e.g., mammals, including humans and non-human primates); or pathogens, such as bacteria and viruses. In some cases, the sample can be, or can be obtained from, cells, tissue, or polynucleotides of a population of such organisms of interest. As another example, the sample can be a microbiome or microbiota. Optionally, the sample is an environmental sample, such as a sample of water, air, or soil.
Samples from an organism of interest, or a population of such organisms of interest, can include, but are not limited to, samples of bodily fluids (including, but not limited to, blood, urine, serum, lymph, saliva, anal and vaginal secretions, perspiration and semen); cells; tissue; biopsies, research samples (e.g., products of nucleic acid amplification reactions, such as PCR amplification reactions); purified samples, such as purified genomic DNA; RNA preparations; and raw samples (bacteria, virus, genomic DNA, etc.). Methods of obtaining target polynucleotides (e.g., genomic DNA) from organisms are well known in the art.

### 2. Target nucleic acid

As used herein, the term "target nucleic acid" (or polynucleotide) or "nucleic acid of interest" refers to any nucleic acid (or polynucleotide) suitable for processing and sequencing by the methods described herein. The nucleic acid may be single-stranded or double-stranded and may include DNA, RNA, or other known nucleic acids. The target nucleic acids may be those of any organism, including but not limited to viruses, bacteria, yeast, plants, fish, reptiles, amphibians, birds, and mammals (including, without limitation, mice, rats, dogs, cats, goats, sheep, cattle, horses, pigs, rabbits, monkeys and other non-human primates, and humans). A target nucleic acid may be obtained from an individual or from a multiple individuals (i.e., a population). A sample from which the nucleic acid is obtained may contain a nucleic acids from a mixture of cells or even organisms, such as: a human saliva sample that includes human cells and bacterial cells; a mouse xenograft that includes mouse cells and cells from a transplanted human tumor; etc. Target nucleic acids may be unamplified or they may be amplified by any suitable nucleic acid amplification method known in the art. Target nucleic acids may be purified according to methods known in the art to remove cellular and subcellular contaminants (lipids, proteins, carbohydrates, nucleic acids other than those to be sequenced, etc.), or they may be unpurified, i.e., include at least some cellular and subcellular contaminants, including without limitation intact cells that are disrupted to release their nucleic acids for processing and sequencing. Target nucleic acids can be obtained from any suitable sample using methods known in the art. Such samples include but are not limited to biosamples such as tissues, isolated cells or cell cultures, bodily fluids (including, but not limited to, blood, urine, serum, lymph, saliva, anal and vaginal secretions, perspiration and semen); and environmental samples, such as air, agricultural, water and soil samples, etc.

Target nucleic acids may be genomic DNA (e.g., from a single individual), cDNA, and/or may be complex nucleic acids, including nucleic acids from multiple individuals or genomes. Examples of complex nucleic acids include a microbiome, circulating fetal cells in the bloodstream of a expecting mother (see, e.g., Kavanagh et al., J. Chromatol. B 878: 1905-1911, 2010), circulating tumor cells (CTC) from the bloodstream of a cancer patient. In one embodiment, such a complex nucleic acid has a complete sequence comprising at least one gigabase (Gb) (a diploid human genome comprises approximately 6 Gb of sequence).

In some cases, target nucleic acids or first complexes are genomic fragments. In some embodiments the genomic fragments are longer than 10kb, e.g., 10-100kb, 10-500kb, 20-300kb, 50-200kb, 100-400kb, or longer than 500 kb. In some cases, target nucleic acids or first complexes are 5,000 to 100,000 Kb in length. The amount of DNA (e.g., human genomic DNA) used in a single mixture may be <10ng, <3ng, <1ng, <0.3ng, or <0.1ng of DNA. In some embodiments, the amount of DNA used in the single mixture may be less than 3,000x, e.g., less than 900x, less than 300x, less than 100x, or less than 30x of haploid DNA amount. In some embodiments, the amount of DNA used in the single mixture may be at least 1x of haploid DNA, e.g., at least 2x, or at least 10 x haploid DNA amount.

Target nucleic acids may be isolated using conventional techniques, for example as disclosed in Sambrook and Russell, Molecular Cloning: A Laboratory Manual*,* cited *supra.* In some cases, particularly if small amounts of the nucleic acids are employed in a particular step, it is advantageous to provide carrier DNA, e.g., unrelated circular synthetic double-stranded DNA, to be mixed and used with the sample nucleic acids whenever only small amounts of sample nucleic acids are available and there is danger of losses through nonspecific binding, e.g., to container walls and the like.

According to some embodiments of the invention, genomic DNA or other complex nucleic acids are obtained from an individual cell or small number of cells with or without purification, by any known method.

Long fragments are desirable for the methods of the present invention. Long fragments of genomic DNA can be isolated from a cell by any known method. A protocol for isolation of long genomic DNA fragments from human cells is described, for example, in Peters et al., Nature 487:190-195 (2012). In one embodiment, cells are lysed and the intact nuclei are pelleted with a gentle centrifugation step. The genomic DNA is then released through proteinase K and RNase digestion for several hours. The material can be treated to lower the concentration of remaining cellular waste, e.g., by dialysis for a period of time (i.e., from 2 -16 hours) and/or dilution. Since such methods need not employ many disruptive processes (such as ethanol precipitation, centrifugation, and vortexing), the genomic nucleic acid remains largely intact, yielding a majority of fragments that have lengths in excess of 150 kilobases. In some embodiments, the fragments are from about 5 to about 750 kilobases in lengths. In further embodiments, the fragments are from about 150 to about 600, about 200 to about 500, about 250 to about 400, and about 300 to about 350 kilobases in length. The smallest fragment that can be used for haplotyping is one containing at least two hets (approximately 2-5 kb); there is no maximum theoretical size, although fragment length can be limited by shearing resulting from manipulation of the starting nucleic acid preparation.

In other embodiments, long DNA fragments are isolated and manipulated in a manner that minimizes shearing or absorption of the DNA to a vessel, including, for example, isolating cells in agarose in agarose gel plugs, or oil, or using specially coated tubes and plates.

A controlled use of a 5' exonuclease (either before or during amplification) can promote multiple replications of the original DNA from a single cell and thus minimize propagation of early errors through copying of copies.

Fragmented DNA from a single cell can be duplicated by ligating an adaptor with single-stranded priming overhang and using an adaptor-specific primer and phi29 polymerase to make two copies from each long fragment. This can generate four cells-worth of DNA from a single cell.

According to one embodiment of the invention, one starts with more long fragments than are needed for sequencing to achieve adequate sequence coverage and tags only a only a portion of the long fragments with a limited number of tag-containing sequences, or tag assemblies - which include many, perhaps hundreds, of copies of one tag sequence - to increase the probability of unique tagging of the long fragments. Non-tagged subfragments lacking introduced sequences that provide primer-binding or capture-oligonucleotides binding and may be eliminated in downstream processing. Such tag assemblies include, for example, end-to-end concatemers of tag-containing sequences created by rolling circle replication (DNA nanoballs), beads to which are attached many copies of the tag-containing sequences, or other embodiments.

According to another embodiment, in order to obtain uniform genome coverage in the case of samples with a small number of cells (e.g., 1, 2, 3, 4, 5, 10, 10, 15, 20, 30, 40, 50 or 100 cells from a microbiopsy or circulating tumor or fetal cells, for example), all long fragments obtained from the cells are tagged.

### 3. Transposition

Any suitable transposon/transposase or transposon/integrase system may be used in the methods in this disclosure. Examples include in vitro Mu transposition (Haapa et al., Nucl. Acids Res., 27:2777-2784, 1999; Savilahti et al., EMBO J. 14:4893-4903, 1995); Tyl (Devine and Boeke, Nucl. Acids Res., 22:3765-3772, 1994; International Patent Application WO 95/23875); Tn7 (Craig, Curr. Topics Microbiol. Immunol. 204:27-48, 1996); Tn 10 and IS 10 (Kleckner et al., Curr. Top. Microbiol. Immunol. 204:49-82, 1996); Mariner (Lampe et al., EMBO J. 15:5470-5479, 1996); Tcl (Vos et al., Genes Dev.,10:755-761, 1996); Tn5 (Park et al., Taehan Misaengmul Hakhoechi 27:381-389, 1992); P element (Kaufman and Rio, Cell 69:27-39, 1992); Tn3 (Ichikawa and Ohtsubo, J. Biol. Chem. 265:18829-18832, 1990); bacterial insertion oligonucleotides (Ohtsubo and Sekine, Curr. Top. Microbiol. Immunol., 204:1-26, 1996); retroviruses (Varmus and Brown, "Retroviruses," in Mobile DNA, Berg and Howe, eds., American Society for Microbiology, Washington, DC, pp.53-108, 1989); and yeast retrotransposons (Boeke, "Transposable elements in Saccharomyces cerevisiae," in Mobile DNA, Berg and Howe, eds., American Society for Microbiology, Washington, DC, pp.53-108, 1989). Other known transposons include, without limitation, Tn5, AC7, Tn5SEQ1, Tn916, Tn951, Tn1721, Tn 2410, Tn1681, Tn1, Tn2, Tn4, Tn6, Tn9, Tn30, Tn101, Tn903, Tn501, Tn1000 (γ6), Tn1681, Tn2901, AC transposons, Mp transposons, Spm transposons, En transposons, Dotted transposons, Ds transposons, dSpm transposons and I transposons. Modified forms of the transposon ends and/or transposases may be used, e.g., a modified Tn5 transposase as in the Nextera^{™} technology (Epicentre Biotechnologies, Madison, Wl). See US20180016571 A1.

Many transposases recognize different insertion oligonucleotides, and therefore it is to be understood that a transposase-based vector will contain insertion oligonucleotides recognized by the particular transposase also found in the transposase-based vector. Transposases and insertion oligonucleotides from eukaryotic transposon-based vectors can be modified and used including. However, non-eukaryotic transposon-based elements reduce the likelihood that a eukaryotic transposase in the recipient organism (e.g., human subject) will recognize prokaryotic insertion oligonucleotides bracketing the transgene.

When in use, transposons are combined with long fragments of DNA (double-stranded or partially double-stranded) and the addition of transposase causes transposition of the insertion oligonucleotide into the long fragments. The transposons carry two transposon ends having sequence complementary to target sequence. The methods of different ways transposition occurs are described in WO 2014/145820. The transposon includes an insertion oligonucleotide, which comprises a single-stranded region for hybridization ("hybridization sequence") as well as a double-stranded mosaic sequence that is recognized by the transposase and enables the transposition reaction. The transposase enzyme has the property of remaining bound to genomic DNA after the transposition event, effectively leaving the transposon-integrated long genomic DNA molecule intact, which can be tagged by the barcodes from the same bead. After barcoding, transposase can be removed by such as treated with SDS, which results in separate nucleic acid fragments.

A transposase catalyzes the random insertion of excised transposons into DNA targets. During cut-and-paste transposition, a transposase makes random, staggered single-stranded breaks in the target DNA and covalently attaches the 3' end of the transferred transposon strand to the 5' end of the target DNA. The transposase/transposon complex inserts an arbitrary DNA sequence at the point of insertion of the transposon into the target nucleic acid. In some cases, transposons that insert randomly into the target nucleic acid sequence are used. Several transposons have been described and use in in vitro transposition systems. For example, in the Nextera^{™} technology (Nature Methods 6, November 2009; Epicentre Biotechnologies, Madison, WI) The entire complex is not necessary for insertion; free transposon ends are sufficient for integration. When free transposon ends are used, the target DNA is fragmented and the transferred strand of the transposon end oligonucleotide is covalently attached to the 5' end of the target fragment. The transposon ends can be modified by addition of desired sequences, such as PCR primer binding sites, bar codes/tags, etc. The size distribution of the fragments can be controlled by changing the amounts of transposase and transposon ends. Exploiting transposon ends with appended sequences results in DNA libraries that can be used in high-throughput sequencing.

The frequency of transposition events is positively correlated to the concentration of the transposons and transposases, that is, in order to generate large genomic fragments, as in the first round of transposition events, the lower concentration of transposon and transposase should be used; and in order to generate smaller genomic fragments, as in the second round of the transposition, a higher concentration of transposon and transposase are used.

The insertion oligonucleotide in the transposon may comprise tags or a sequence, which can be used for hybridizing to other nucleic acids for further analysis. In some embodiments, the insertion oligonucleotide comprises a sequence that can hybridize to a splint oligo, which also hybridizes to a capture oligonucleotide attached to beads, as described below.

### 4. Creating nicks using nickase

Various embodiments in the method requires introduction of nicks in nucleic acid fragments before they are barcoded by the beads. As one of the alternative approaches of introducing nicks by transposition, a nickase can be used to create nicks in nucleic acid fragments. In some embodiments, the nickase nicks DNA at its recognition sequence. In some embodiments, the nickase nicks DNA at random positions. Non-limiting examples of nickases include Nb.BsrDI, Nb.Bsml, Nt.BbvCl, Nb.Bbv, Nb.Btsl or Nt.BstNBI, etc. The nicks created by the nickase are widened by an enzyme such as klenow fragment and the resulted fragments are ligated by a ligase to capture oligonucleotide adaptors immobilized on the beads. The capture oligonucleotide adaptors can be double-stranded or partially double-stranded. Over time, nicking continues and more gaps are opened for more adapters to ligate into gaps. In some embodiments, the nickase is used at low concentration, Klenow fragment at medium concentration, and ligase at high concentration to allow low opening of nicks to gaps and rapid ligation, which locks DNA fragments to beads.

Each of the capture oligonucleotide adapters on the bead is attached to the bead at one end through one or both strands, and has a blunt end at the other end. One strand of the adapter comprises a capture oligonucleotide and the other strand is complementary to the capture oligonucleotide. The capture oligonucleotide may comprise the various components described in this application, e.g., a primer binding sequence, promoter sequence, barcode, and/or UMI. In some embodiments, the 5' terminus of the capture oligonucleotide is attached to the bead, and the 5' of its complementary strand can be ligated to the 3' terminus of the DNA fragments through 3' branch ligation (FIG. 27A). In some embodiments, the 3' terminus of the capture oligonucleotide is attached to the bead, and the 5' of capture oligonucleotide strand can be ligated to the 3' of the DNA fragments through 3' branch ligation (FIG. 27B).

### 4. Barcode

According to one embodiment, a barcode-containing sequence is used that has two, three or more segments of which, one, for example, is the barcode sequence. For example, an introduced sequence may include one or more regions of known sequence and one or more regions of degenerate sequence that serves as the barcode(s) or tag(s). The known sequence (B) may include, for example, PCR primer binding sites, transposon ends, restriction endonuclease recognition sequences (e.g., sites for rare cutters, e.g., Not I, Sac II, Mlu I, BssH II, etc.), or other sequences. The degenerate sequence (N) that serves as the tag is long enough to provide a population of different-sequence tags that is equal to or, preferably, greater than, the number of fragments of a target nucleic acid to be analyzed.

According to one embodiment, the barcode-containing sequence comprises one region of known sequence of any selected length. According to another embodiment the barcode-containing sequence comprises two regions of known sequence of a selected length that flank a region of degenerate sequence of a selected length, i.e., BₙNₙBₙ, where N may have any length sufficient for tagging long fragments of a target nucleic acid, including, without limitation, N = 10, 11, 12, 13, 14, 15, 16, 17, 18, 19 or 20, and B may have any length that accommodates desired sequences such as transposon ends, primer binding sites, etc. For example, such an embodiment may be B₂₀N₁₅B₂₀.

In one embodiment, a two- or three-segment design is utilized for the barcodes used to tag long fragments. This design allows for a wider range of possible barcodes by allowing combinatorial barcode segments to be generated by ligating different barcode segments together to form the full barcode segment or by using a segment as a reagent in oligonucleotide synthesis. This combinatorial design provides a larger repertoire of possible barcodes while reducing the number of full-size barcodes that need to be generated. In further embodiments, unique identification of each long fragment is achieved with 8-12 base pair (or longer) barcodes.

In one embodiment, two different barcode segments are used. A and B segments are easily be modified to each contain a different half-barcode sequence to yield thousands of combinations. In a further embodiment, the barcode sequences are incorporated on the same adapter. This can be achieved by breaking the B adaptor into two parts, each with a half barcode sequence separated by a common overlapping sequence used for ligation. The two tag components have 4-6 bases each. An 8-base (2 x 4 bases) tag set is capable of uniquely tagging 65,000 sequences. Both 2x5 base and 2x6 base tags may include use of degenerate bases (i.e., "wild-cards") to achieve optimal decoding efficiency.

In further embodiments, unique identification of each sequence is achieved with 8-12 base pair error correcting barcodes. Barcodes may have a length, for illustration and not limitation, of from 5-20 informative bases, usually 8-16 informative bases.

### 5. Barcoded beads

The first beads and the second beads are barcoded by oligonucleotides having barcodes that are immobilized on the beads. Each bead carries many copies of a unique barcode sequence which is transferred to the sub-fragments of each long DNA molecule.

The beads used may have a diameter in the range of 1-20 um, alternatively 2-8 um, 3-6 um or 1-3 um, e.g., about 2.8 µm. For example, the spacing of barcoded oligonucleotides on the beads is can at least 1, at least 2, at least 3, at least 4, at least 5, at least 6 or at least 7 nm. In come embodiments the spacing is less than 10nm (e.g., 5-10nm), less than 15nm, less than 20nm, less than 30 nm, less than 40 nm, or less than 50 nm. In some embodiments, the number of different barcodes used per mixture may be >1M, >10M, >30M, >100M, >300M, or >1B. As discussed below, a very large number of barcodes may be produced for use in the invention, e.g., using methods described herein. In some embodiments, the number of different barcodes are used per mixture may be >1M, >10M, >30M, >100M, >300M, or >1B and they are sampled from a pool of at least 10-fold greater diversity (e.g. from >10M, >0.1B, 0.3B, >0.5B, >1B, >3B, >10B different barcodes on beads.) In some embodiments, the number of barcodes per bead is between 100k to 10M, e.g., between 200k and 1M, between 300k and 800k, or about 400k.

In some embodiments, the barcode region is about 3-15 nucleotides in length, e.g., 5-12, 8-12, or 10 nucleotides in length. In some cases, each barcode of the barcode region is about 3-12 nucleotides in length, or 3-5 nucleotides in length. Thus, a barcode, whether sample barcode, cell barcode or other barcode can be 3, 4, 5, 6, 7, 8, 9, 10, 11, 12, 13, 14, 15 or more nucleotides in length. In one particular example, each barcode region comprises three barcodes, each consisting of 10 bases, and the three barcodes are separated by 6 bases of common sequence.

Barcoded beads are transferred to the target sequence. In some embodiments, the transfer occurred at regular intervals through ligation of the 3' terminus of the capture adaptor to the 5' terminus of the transposon-inserted hybridization sequence mediated by a bridge or splint (terms used interchangeably, one example of the splint oligonucleotides is shown as ④ in Figure 2) oligonucleotide with a first region complementary to the capture adaptor and a second region complementary to the hybridization sequence. Beads are collected and DNA/transposase complexes are disrupted producing sub-fragments less than 1 kb in size. In some embodiments, the transfer occurred by direction ligation to the target DNA.

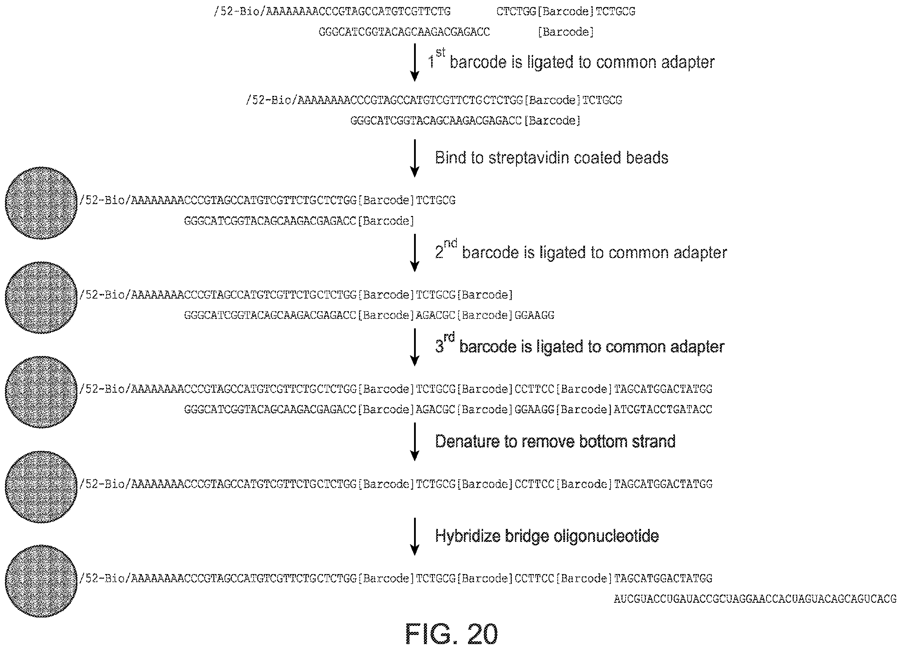

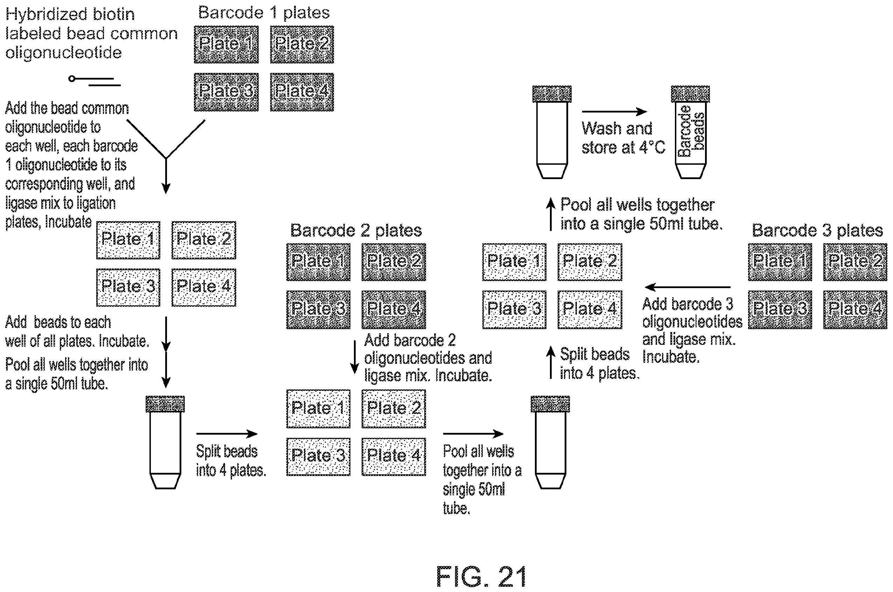

In some embodiments, the barcoded beads are constructed through a split and pool ligation-based strategy using three sets of double-stranded barcode DNA molecules. In some embodiments, each set of double-stranded barcode DNA molecules consists of 10 base pairs and the three sets are different in nucleic acid sequence. An exemplary method of the split and pool ligation to produce the barcoded beads is described in the PCT Pub. No. WO 2019/217452. Figures 20 and 21 also illustrate the methodology of the split and pool method. A common adapter sequence comprising a PCR primer annealing site was attached to Dynabeads^{™} M-280 Streptavidin (ThermoFisher, Waltham, MA) magnetic beads with a 5' dual-biotin linker. Three sets 1,536 of barcode oligos containing regions of overlapping sequence were constructed by Integrated DNA Technologies (Coralville, IA). Ligations were performed in 384 well plates in a 15 µL reaction containing 50 mM Tris-HCl (pH 7.5), 10 mM MgCl₂, 1 mM ATP, 2.5% PEG-8000, 571 units T4 ligase, 580 pmol of barcode oligo, and 65 million M-280 beads. Ligation reactions were incubated for 1 hour at room temperature on a rotator. Between ligations beads were pooled into a single vessel through centrifugation, collected to the side of the vessel using magnet, and washed once with high salt wash buffer (50 mM Tris-HCl (pH 7.5), 500 mM NaCl, 0.1 mM EDTA, and 0.05% Tween 20) and twice with low salt wash buffer (50 mM Tris-HCl (pH 7.5), 150 mM NaCl, and 0.05% Tween 20). Beads were resuspended in 1X ligation buffer and distributed across 384 wells plates and the ligation steps were repeated.

Certain "barcodes" referred to herein are "tripartate barcodes." Tripartate refers to their structure and/or to their synthesis. As shown in Fig. 20 the tripartate barcodes may be synthesized by successive ligations of shorter (e.g., 4-20 nucleotide) sequences. In one embodiment the shorter barcodes are 10 bases in length. As shown in the figure, an exemplary structure comprised CS1-BC1-CS2-BC2-CS3-BC3-CS4 wherein CS is a constant sequence present on all capture adaptors and the BC sequences are diverse 10 base barcode as discussed here. The tripartate barcode can be constructed using partially double-stranded oligonucleotides with the structure CSa-BC-CSb annealed to a shorter oligonucleotidethat is the complement of BC (i.e., BC') as shown in the figures.

Examples not encompassed by the wording of the claims include a composition comprising beads with capture oligonucleotides comprising clonal barcodes attached, where the composition comprises more than 3 billion different barcodes and where the barcodes are tripartate barcodes with the structure 5'- CS1-BC1-CS2-BC2-CS3-BC3-CS4. In some embodiments CS1 and CS4 are loner than CS2 and CS3. In some embodiments CS2 and CS3 are 4-20 bases, CS1 and CS4 are 5 or 10 to 40 bases, e.g., 20-30, and the BC sequences are 4-20 bases (e.g., 10 bases) in length. In some embodiments CS4 is complementary to a splint oligonucleotide. In some embodiments the composition comprises bridge oligonucleotides. In some embodiments the composition comprises bridge oligonucleotides, beads comprising a tripartate barcode as discussed above, and genomic DNA comprising hybridization sequences with a region complementary to the bridge oligonucleotides.

Another source of clonal barcodes such as a bead or other support associated with multiple copies of tags can be prepared by emulsion PCR or CPG (controlled-pore glass) or chemical synthesis other particles with copies of an adapted-barcode prepared by. A population of tag-containing DNA sequences can be PCR amplified on beads in an water-in-oil (w/o) emulsion by known methods. See, e.g., Tawfik and Griffiths Nature Biotechnology 16: 652-656 (1998); Dressman et al., Proc. Natl. Acad. Sci. USA 100:8817-8820, 2003; and Shendure et al., Science 309:1728-1732 (2005). This results in many copies of each single tag-containing sequence on each bead.

Another method for making a source of clonal barcodes is by oligonucleotide synthesis on micro-beads or CPG in a "mix and divide" combinatorial process. Using this process one can create a set of beads each having population of copies of a barcode. For example, to make all B₂₀N₁₅B₂₀ where each of about 1 billion is represented in ^{~}1000+ copies on each of 100 beads, on average, one can start with ^{~}100 billion beads, synthesize B₂₀ common sequence (adaptor) on all of them and then split them in 1024 synthesis columns to make a different 5-mer in each, then mix them and then split them again in 1024 columns and make additional 5-mer, and then repeat that once again to complete N15, and then mix them and in one big column synthesize the last B₂₀ as a second adaptor. Thus, in 3050 syntheses one can make the same "clonal-like" sets of barcodes as in one big emulation PCR reaction with ^{~}1000 billion beads (1¹² beads) because only 1 in 10 beads will have a starting template (the other 9 would have none) to prevent having two templates with different barcode per bead.

An exemplary process for the barcode sequence assembly is shown in FIG. 21.

### 6. UMI

In various embodiments, unique molecular identifiers (UMIs) are used to distinguish individual DNA molecules from one another. For example, UMIs are used to distinguish among the capture oligonucleotides that are immobilized on the first beads (e.g., Scheme I step 2). The collection of adapters is generated, each having a UMI, and those adapters are attached to fragments or other source DNA molecules to be sequenced, and the individual sequenced molecules each has a UMI that helps distinguish it from all other fragments. In such implementations, a very large number of different UMIs (e.g., many thousands to millions) may be used to uniquely identify DNA fragments in a sample.

I The UMI is at a length that is sufficient to ensure the uniqueness of each and every source DNA molecule. In some embodiments, the unique molecular identifier is about 3-12 nucleotides in length, or 3-5 nucleotides in length. In some cases, each unique molecular identifier is about 3-12 nucleotides in length, or 3-5 nucleotides in length. Thus, a unique molecular identifier can be 3, 4, 5, 6, 7, 8, 9, 10, 11, 12, 13, 14, 15, 16, 17, 18, or more nucleotides in length.

### 7. Amplification

In some embodiments, a subfragment or fragment produced in the method steps are amplified. Such amplification methods include without limitation: multiple displacement amplification (MDA), polymerase chain reaction (PCR), ligation chain reaction (sometimes referred to as oligonucleotide ligase amplification OLA), cycling probe technology (CPT), strand displacement assay (SDA), transcription mediated amplification (TMA), nucleic acid sequence based amplification (NASBA), rolling circle amplification (RCR) (for circularized fragments), and invasive cleavage technology. Amplification can be performed after fragmenting or before or after any step outlined herein.

### 8. Reverse transcription

Reverse transcription is mediated by a reverse transcriptase, which use RNA as template to synthesize cDNA that is complementary to the RNA template. Reverse transcriptases typically have RNA dependent DNA polymerase activity and RNase H activity. In some cases, the reverse transcriptase also possess DNA-dependent DNA polymerase activity. Reverse transcription typically involves a number of steps: 1. In the presence of an annealed primer, reverse transcriptase binds to an RNA template and initiates the reaction; 2. RNA-dependent DNA polymerase activity synthesizes the complementary DNA strand, incorporating dNTPs; 3. RNase H activity degrades the RNA template of the DNA:RNA complex; 4. DNA-dependent DNA polymerase activity (if present) recognizes the single-stranded cDNA as a template, uses an RNA fragment as a primer, and synthesizes the second-strand cDNA to form double-stranded cDNA.

Non-limiting examples of reverse transcriptases include; HIV-1 reverse transcriptase from human immunodeficiency virus type 1; M-MLV reverse transcriptase from the Moloney murine leukemia virus; AMV reverse transcriptase from the avian myeloblastosis virus; Telomerase reverse transcriptase that maintains the telomeres of eukaryotic chromosomes.

### 9. 3' Branch ligation

3' branch ligation, involves the covalent joining of the 5' phosphate from a blunt-end adapter (donor DNA) to the 3' hydroxyl end of a duplex DNA acceptor at 3' recessed strands, gaps, or nicks. In contrast to conventional DNA ligation, 3' branch ligation does not require complimentary base pairing. 3' branch ligation is described in the PCT Pub. No. WO 2019/217452; US Pat. Pub. US2018/0044668 and International Application WO 2016/037418, US Pat. Pub. 2018/0044667, as well as Wang et al., June 29, 2018, http://dx.doi.org/10.1101/357863. Using this method, it is theoretically possible to amplify and sequence all sub-fragments of a captured genomic molecule. Thus, 3' branch ligation has broad range of molecular applications, including, e.g., attaching adaptors to DNA or RNA during NGS library preparation.

In addition, this ligation step enables a sample barcode to be placed adjacent to the genomic sequence for sampling multiplexing. The benefit of using these adapters for sample barcoding is that the barcode can be placed adjacent to the genomic DNA so that the same primer can be used to sequence the barcode and the genomic DNA and no additional sequencing primer is required to read the barcode. Sample barcoding allows preparations from multiple samples to be pooled before sequences, and distinguished by the barcode. 3' branch ligation adapters can be synthesized in 96, 384, or 1536 plate format, with each well containing many copies of the adapter carrying the same barcode and each barcode being different between wells. After capture on beads these adapters can be used for ligation in 96, 384, or 1536 plate format.

After this ligation step, PCR is performed and the library is ready to enter any standard next generation sequencing (NGS) workflow. It will be appreciated that PCR (or other amplification) can be carried out using a first primer that hybridizes to a site on the capture oligonucleotide or its complement (see Fig. W1A) and a second primer that hybridizes to a site on the 3' branch ligation adapter or its complement. In the case of BGISEQ-500, the library is circularized as previously described (17). From single-stranded circles DNA nanoballs are made and loaded onto patterned nanoarrays (17). These nanoarrays are then subjected to combinatorial probe-anchor synthesis (cPAS) based sequencing on the BGISEQ-500 (18-20). After sequencing, barcode sequences are extracted. Mapping the read data by unique barcode shows that most reads with the same barcode are clustered in a region of the genome corresponding to the length of DNA used during library preparation (Figure 1B). A detailed description of this method, as well as a protocol for making the beads is described in EXAMPLES 1 and 2.

### 10. Transpositions using transposons containing positional barcodes

In some cases, transposons inserted into a target nucleic acid according to any of the embodiments above may carry a unique, positional barcode. This positional barcode denotes the position of each transposon relative to other transposons. This positional barcode can be useful in assembling sequence reads for regions containing sequence repeats. FIG. 23 exemplifies a genomic region containing four sequence regions, I, II, III, and IV. In this region, I, II and IV are repeats, III differs from I, II, or IV by only one nucleotide. In the absence of positional barcodes, it would be difficult to determine whether a particular sequence read belongs to region I, II, III, or IV. Using positional barcodes would avoid this problem, by associating the sequence read to a particular positional barcode that is unique to regions I, II, III, or IV, the sequence read can be assigned to a specific region. Thus, using transposons carrying positional barcodes can facilitate ordering repeat sequences, and together with the barcodes introduced by the beads, the sequence of long-range repeat regions can be efficiently determined.

Inserting transposons with positional barcode into a target nucleic acid (e.g., a genomic DNA molecule) may be achieved by contacting one or more transposon scaffolds with the target nucleic acid molecule. The transposon scaffold holds transposons with unique positional barcodes at predetermined spacing, said spacing dictating the size of the intervals after the transposons are inserted into the target nucleic acid molecule. The embodiments of these methods are further described as below.

### Scaffold

As described herein, an insertion scaffold is a composite structure comprising a scaffold and adaptors that are anchored thereon. The adaptors are double-stranded or partially double-stranded. The scaffold can be any suitable materials that can hold the adaptors at desired spacing. Non-limiting examples of scaffolds include nucleic acids, proteins, carbohydrate molecules, and other long chemical structures soluble in aqueous solutions. In some embodiments, the scaffold is a single-stranded nucleic acid molecule, e.g., a single-stranded DNA derived from a known plasmid, such as PUC 19. The size of the scaffold nucleic acid molecule may vary. In some embodiments, the size of the scaffold may range from 1 to 50 kb, e.g., from 1 to 30 kb, from 2-30 kb, or from 5 to 10 kb. The adaptors contain positional barcodes and/or scaffold barcodes, which can be inserted into target DNA fragments. In some embodiments, the insertion is by 3'branch ligation. The adaptors can be transposons. The adaptors containing positional barcodes and/or scaffold barcodes, which are inserted into the DNA fragments by transposition. However, one skilled in the art would appreciate that any double-stranded or partially double-stranded adaptors can be produced having the various positional barcodes and/or scaffold barcodes, and these adaptors can be anchored to scaffolds in the same manner as transposons. These adaptors can be inserted into the target DNA fragments through, e.g., 3' branch ligation, as described above.

### Sequence features of the transposons

### Scaffold hybridization sequence in the transposons

In some embodiments, adaptors (e.g., transposons) used in each scaffold comprise specific scaffold hybridization sequences, i.e., sequences that are complementary to different regions in the scaffold, such that when the adaptors (e.g., transposons) are mixed with the scaffold, the adaptors (e.g., transposons) are anchored to the scaffold via these hybridization sequences at desired spacing. The scaffold hybridization sequences are designed to be cleavable, so that after being incorporated into the target nucleic acid by transposition, the hybridization sequences can be cleaved to dissociate adaptors (e.g., transposons) from the scaffold. After the cleavage of the scaffold hybridization sequence, the scaffold barcode and the positional barcode remain in the adaptors (e.g., transposons) that are inserted into the target nucleic acid molecule. The scaffold that has been dissociated can then be removed from the reaction by e.g., washing.

### Scaffold barcode

Adaptors (e.g., transposons) may comprise scaffold barcodes, which are unique to each transposon scaffold. Adaptors (e.g., transposons) in the same transposon scaffold share the same scaffold barcode and adaptors (e.g., transposons) from different transposon scaffold have different scaffold barcodes. These scaffold barcodes allow grouping sequence information that correspond to the same scaffold. FIG. 23 shows an exemplary design of a number of transposon scaffolds, S1, S2, ..., Sn. All adaptors (e.g., transposons) in the S1 scaffold share the same S1 scaffold barcode, and all adaptors (e.g., transposons) in the S2 scaffold share the same S2 scaffold, etc. The scaffold barcodes for different transposon scaffolds, S1, S2, S3, ..., Sn, are all distinguishable.

### Positional barcode

Each transposon in the same scaffold may carry a unique positional barcode, and different adaptors (e.g., transposons) within the same scaffold carry different positional barcodes. FIG. 23 shows an example of transposon scaffolds comprising adaptors (e.g., transposons) comprising positional barcodes. For example, adaptors (e.g., transposons) that are held by scaffold S1 have the same S1 scaffold barcode but have different positional barcodes. 1 and 2 are positional barcodes on the first transposon, 3 and 4 are positional barcodes on the second transposon, 5 and 6 are positional barcodes on the third transposon, 7 and 8 are positional barcodes on the fourth transposon, etc.

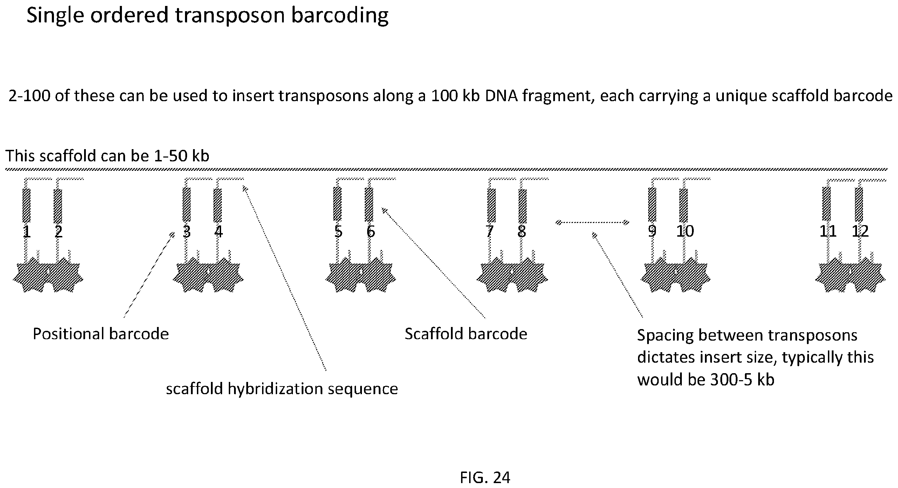

In some cases, the two insertion oligonucleotides of the same transposon may have the same positional barcodes; for example, position barcodes 1 and 2 in FIG. 24 may be identical. In some cases, the two insertion oligonucleotides of the same transposon may have different barcode sequences; for example, position barcodes 1 and 2, may be different.

### Producing insertion scaffolds

Adaptors (e.g., transposons) having one or more of the sequence features described above, e.g., the positional barcodes, scaffold barcodes, and/or scaffold hybridization sequences, can be anchored on the scaffold via chemistry that is suitable for the scaffold. In some cases, the scaffold is a single-stranded nucleic acid, and adaptors (e.g., transposons) are hybridized to the scaffold at specific locations via scaffold hybridization sequences comprised in the adaptors (e.g., transposons). In some cases, the sequence of the single-stranded nucleic acid scaffold is known, so that the scaffold hybridization sequences of the adaptors (e.g., transposons) can be designed to ensure anchorage of adaptors (e.g., transposons) at desired locations at desired frequencies. In another words, both the positions of the individual adaptors (e.g., transposons) and the spacing between adjacent adaptors (e.g., transposons) can be predetermined. In some cases, adaptors (e.g., transposons) are anchored along the scaffold with spacing that range from 300 bases-5kb, e.g., 400 bases-4kb, 500 bases-2kb, or about 500 bases-1kb. The spacing between adjacent adaptors (e.g., transposons) in the same scaffold need not to identical, although in some embodiments, the spacing between adjacent adaptors (e.g., transposons) in the same scaffold are the same. The number of adaptors (e.g., transposons) per scaffold may also vary, depending on the size of the scaffold and the desired spacing among the adaptors (e.g., transposons). In some cases, the number of adaptors (e.g., transposons) per scaffold may range from 3 to 20, e.g., from 4 to 15, or from 5 to 10. FIG. 24 shows an exemplary transposon scaffold with six adaptors (e.g., transposons) hybridized to a single-stranded DNA molecule the sequence of which is known.

In certain embodiments, the scaffold is a protein or substrate that is modified to contain chemically modified sites that can be used to attach, either covalently or non-covalently, attach adaptors (e.g., transposons) to the substrate. These types of transposon scaffolds can also be designed to have adaptors (e.g., transposons) anchored to the scaffold at desired locations with desired spacing, similar to the transposon scaffold where the scaffold is a nucleic acid molecule.

### Transpositions with transposon scaffolds

The number of transposon scaffolds used for transposing each target nucleic acid may vary. It may be determined based on the size of the target nucleic acid and the size of each scaffold to ensure sufficient coverage of the target nucleic acid. In some embodiments, a sufficient number of scaffolds are used so that the total length of the scaffolds is equal to or greater than the length of the target nucleic acid.

Transposon scaffolds can be mixed with target nucleic acid (genomic DNA), in the presence of a transposase, under conditions that are suitable for transposition. The adaptors (e.g., transposons) are then inserted into the target nucleic acid. See FIG. 25 A. As described above, after the transposition, the scaffold hybridization sequences of the incorporated adaptors (e.g., transposons) can be cleaved to remove the scaffolds. In this way, the position information of the adaptors (e.g., transposons) as they were in the scaffold are preserved when they are incorporated into the target nucleic acid.

### Adaptors (e.g., transposons) with positional barcodes

Adaptors (e.g., transposons) with positional barcodes and scaffold barcode can be used in any of the schemes described above. In some cases, one round of transposition with the adaptors (e.g., transposons) having positional barcodes is performed. As exemplified in FIG. 25A, after the transposition, the nucleic acid fragments are captured using stLFR beads and barcoded as described above. The transposases are then removed to release the individual fragments that were held together by the transposases. The fragments can then be amplified using methods well known in the art. In some cases, these fragments are circularized and amplified using RCR. In some embodiments, the fragments are captured using stLFR beads and co-barcoded as described above. The transposases are then removed and the individual fragments are amplified by RCR

In some embodiments, two rounds of transpositions with adaptors (e.g., transposons) having positional barcodes can be carried out. Typically, after the first round of transposition, fragments defined by adjacent adaptors (e.g., transposons) (first subfragments) are amplified and a second round of transposition is performed on the amplified first subfragments. Depending on methods of amplification, the amplified first subfragments can either be double-stranded or single-stranded. If the amplification first subfragments are double-stranded DNA, they can be processed directly with a second round of transposition. If the amplified first subfragments are single-stranded DNA, they are converted to double-stranded DNA before a second round of transposition is performed. The second round of transposition produces second complexes, which can then be captured on stLFR beads and co-barcoded. After removal of transposases, the subfragments can be processed for sequencing.

FIG. 25A and 25B show an exemplary embodiment of the invention in which two rounds of transposition with adaptors (e.g., transposons) having positional barcodes are performed. FIG. 25A shows that the first round of transposition results in formation of 3-5 kb sized molecules (first subfragments), which are captured on stLFR beads, co-barcoded, circularized, and amplified by RCR. The amplification products are converted to double-stranded DNA. FIG. 25B shows that second round of transposition is performed on the 3-5 kb double-stranded DNA produced from the first round transposition. This produces 500 bp fragments between the adjacent adaptors (e.g., transposons) (second subfragments), and each of the 500 bp fragments can be sequenced to generate sequence reads.

In some embodiments, the positional barcodes in the adaptors (e.g., transposons) used in the second round of transposition are different from those in the adaptors (e.g., transposons) used in the first round of transposition. In some embodiments, the positional barcodes in the adaptors (e.g., transposons) used in the second round of transposition are the same as some of those in the adaptors (e.g., transposons) used in the first round of transposition.

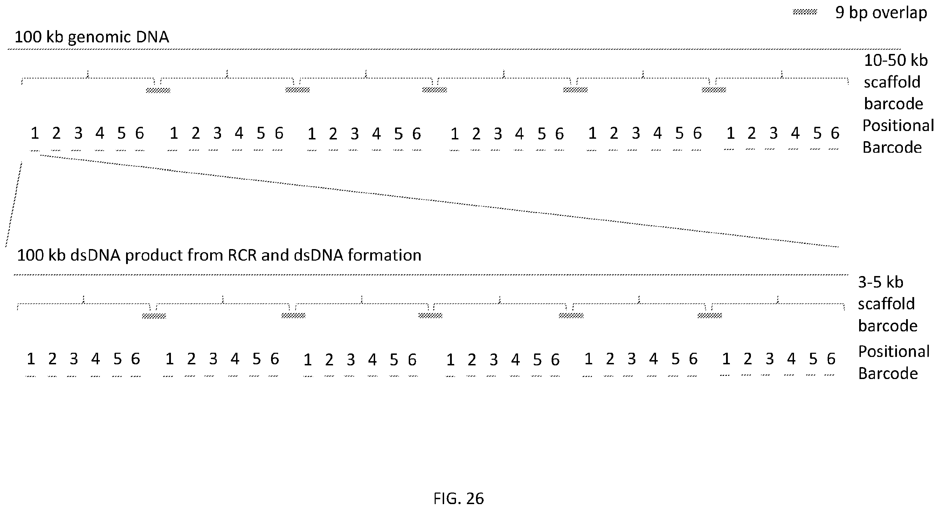

The insertion of each transposon results in a 9 bp overlap between adjacent subfragments, in both first round and second round transpositions, as illustrated in FIG. 26. This information can be used to order subfragments between scaffolds, as further described below.

### Use of positional barcodes and scaffold barcodes for de novo sequencing

The method described in this section, using transposons carrying scaffold barcodes and positional barcodes, provides important information regarding the relative position of each sequence read from sequencing fragments and improve the accuracy and efficiency of de novo sequencing of long target nucleic acid molecule. The positional barcodes can be used to order the sequences within each scaffold. In addition, each transposon insertion results in an overlap between adjacent subfragments (e.g., the first subfragments produced from the first round of transposition and the second subfragments produced from the second round of transposition). For example, transposition of Tn5 typically results in a 9 bp sequence overlap. The sequence of the overlap is unique in the context of typical genomic DNA fragments and the overlap can be readily located in sequencing results. The sequence overlap, in combination with the scaffold barcodes, can be used to order sequences associated with transposons from different scaffolds. For example, a result of two sequences sharing a sequence overlap but carrying different scaffold barcodes, indicates that these two sequences are associated with two transposons from two adjacent scaffolds and the two transposons were located the end of their respective scaffolds.

In scenarios where two rounds of transposition with transposons having the above features, and each round of transposition is also followed by stLFR bead capturing and co-barcoding, sequencing of the resultant second subfragments, such as the 500 bp fragments in FIG. 25B, can generate a wealth of sequence information at many layers. For example, these second subfragments may comprises 1) a first scaffold barcode, 2) a first positional barcode, 3) a 9 bp overlap resulted from the first transposition, 4) a second scaffold barcode, 5) a second positional barcode, 6) a first bead barcode, 7) a second bead barcode, 8) a 9 bp overlap resulted from the second transposition, or combinations thereof. All of these features can be detected in sequence reads and any of combination of these features can greatly improve the accuracy and efficiency of de novo sequencing.

FIG. 26 illustrates an example of de novo sequencing of a 100 kb genomic DNA molecule. Two rounds of transposition are performed and the transposed fragments after each round are captured using stLFR beads and barcoded. The resultant genomic fragments after the second round of transposition and capturing are sequenced. The sequence reads from second subfragments are ordered based on the position barcodes and scaffold barcodes on the second transposons, and the overlap resulted from the transposition to construct the sequence of each first subfragments. The sequence information of multiple first subfragments are then ordered using positional barcodes in the transposons in the first round of transposition to construct the sequence of the first complexes, e.g., the 100 kb genomic DNA molecule.

### 11. Sequencing

The methods described herein can be used as a pre-processing step for sequencing diploid genomes using any sequencing method known in the art, including for example without limitation, polymerase-based sequencing-by-synthesis (e.g., HiSeq 2500 system, Illumina, San Diego, CA), ligation-based sequencing (e.g., SOLiD 5500, Life Technologies Corporation, Carlsbad, CA), ion semiconductor sequencing (e.g., Ion PGM or Ion Proton sequencers, Life Technologies Corporation, Carlsbad, CA), zero-mode waveguides (e.g., PacBio RS sequencer, Pacific Biosciences, Menlo Park, CA), nanopore sequencing (e.g., Oxford Nanopore Technologies Ltd., Oxford, United Kingdom), pyrosequencing (e.g., 454 Life Sciences, Branford, CT), or other sequencing technologies. Some of these sequencing technologies are short-read technologies, but others produce longer reads, e.g., the GS FLX+ (454 Life Sciences; up to 1000 bp), PacBio RS (Pacific Biosciences; approximately 1000 bp) and nanopore sequencing (Oxford Nanopore Technologies Ltd.; 100 kb). For haplotype phasing, longer reads are advantageous, requiring much less computation, although they tend to have a higher error rate and errors in such long reads may need to be identified and corrected according to methods set forth herein before haplotype phasing.

According to one embodiment, sequencing is performed using combinatorial probe-anchor ligation (cPAL) as described, for example, in US 20140051588, U.S. 20130124100.

Exemplary methods for calling variations in a polynucleotide sequence compared to a reference polynucleotide sequence and for polynucleotide sequence assembly (or reassembly), for example, are provided in U.S. patent publication No. 2011-0004413, (App. No. 12/770,089). See also Drmanac et al., Science 327,78-81, 2010 and application No. 61/623,876, entitled "Identification Of DNA Fragments And Structural Variations"; App. No. 13/649,966, published as US Pat. Pub. 2013-0096841; and App. No. 13/447,087, entitled "Processing and Analysis of Complex Nucleic Acid Sequence Data" published as US Pat. Pub. 2013/0124100.

### 12. Compositions

Also provided is a nucleic acid complex comprising a plurality of insertion scaffolds and target nuclear acid fragment. The plurality of insertion scaffolds are hybridized to the target nuclear acid fragment and each of a plurality of insertion scaffolds comprises a plurality of double-stranded or partially double-stranded adaptors and a scaffold. The adaptors are anchored to the scaffold and separated by predetermined spacing. Each adaptor in each of the insertion scaffolds carries a unique positional barcode and all adaptors in the same insertion scaffold share a common scaffold barcode, and adaptors in different insertion scaffolds have different scaffold barcodes.

In some approaches, one or more of the adaptors are transposons.

Also provided is a composition comprising (1) a population of beads comprising clonal copies of capture oligonucleotides attached thereon and (2) one of more insertion scaffold or one or more nucleic acid complexes above. Each bead comprises a plurality of capture oligonucleotides with the same barcode, and different beads in the population comprise different barcodes.

Also provided is a reaction mixture that is in a single vessel, wherein the reaction mixture comprises a plurality of insertion scaffolds disclosed herein and a plurality of nucleic acid fragments. The reaction mixture may further comprises one or more of
i) an exonuclease;
ii) a DNA polymerase;
iii) a Uracil-DNA Glycosylase;
iv) a ligase;
iv) a 3' Branch Ligation Adaptor, and
v) a transposase.

Also provided is an array comprising a plurality of the nucleic acid complexes described above.

## Claims

1. A method for preparing a sequencing library for sequencing a target nucleic acid comprising:
(a) providing (i) single-stranded DNA circles of the target nucleic acid with one or more copies of unique molecular identifier (UMI) and (ii) a plurality of first beads,
wherein each first bead comprises a plurality of first capture oligonucleotides,
wherein each first capture oligonucleotide comprises a first barcode comprising a first barcode sequence,
wherein all first capture oligonucleotides immobilized on the same individual bead comprise the same first barcode sequence, and a majority of beads have different first barcode sequences,
(b) amplifying the single-stranded DNA circles from (a) by rolling circle amplification to produce a plurality of single-stranded concatemers that comprise at least five copies of a single-stranded fragment,
(c) converting the single-stranded concatemers into double-stranded or partially double-stranded DNA molecules,
(d) introducing staggered single-stranded breaks to the DNA molecules in (c), thereby generating first complexes, each comprising a plurality of first subfragments from one of the DNA molecules in (c),
(e) associating at least some of the first subfragments in the first complexes with first capture oligonucleotides immobilized on a plurality of individual first beads, wherein each individual first bead comprises a plurality of the first capture oligonucleotides, thereby providing barcoded first subfragments.

2. The method of claim 1, wherein at least some first subfragments are linked to first insertion oligonucleotides, and wherein step (e) further comprises:
(1) ligating the first capture oligonucleotides to the first insertion oligonucleotides,
or
(2) hybridizing the first capture oligonucleotides to the first insertion oligonucleotides and then extending the insertion oligonucleotides by a DNA polymerase to incorporate first barcodes.

3. The method of claim 1, wherein step (e) is performed in a single mixture, wherein the number of first beads is greater than the number of target nucleic acid fragments in the single mixture, and wherein each first bead comprises multiple copies of the first capture oligo, immobilized thereon.

4. The method of claim 3 wherein first insertion oligonucleotides are added by ligation or by synthesis to at least some first subfragments in step (d), and wherein step (e) further comprises:
(1) ligating the first capture oligonucleotides to the first insertion oligonucleotides,
or
(2) hybridizing first capture oligonucleotides to the first insertion oligonucleotides and then extending the insertion oligonucleotides by a DNA polymerase to incorporate first barcodes.

5. The method of claim 1, wherein:
(i) each first bead comprises multiple copies of the first capture oligonucleotide, wherein the first capture oligonucleotide is hybridized to a complementary oligonucleotide to form a partially double-stranded first capture oligonucleotide,
wherein the method further comprises step (f) comprising
(1) ligating the first capture oligonucleotide to each of at least some of the first subfragments by 3' branch ligation, or
(2) ligating the complementary oligonucleotide to each of at least some of the first subfragments by 3' branch ligation, and extending the complementary oligonucleotide to incorporate the first barcode sequence; or
(ii) the staggered single-stranded breaks are introduced to the DNA molecules by nickase.

6. The method of any of the claims 1-5, wherein the first capture oligonucleotides further comprise a promoter sequence, and wherein the method further comprises amplifying at least a portion of the barcoded first subfragments by
(1) transcribing the barcoded first complexes to generate RNA transcripts,
(2) reversely transcribing the RNA transcripts using a primer annealing to the promoter sequence to generate cDNA strands of the barcoded first complexes,
(3) circularizing the cDNA to produce circularized cDNA strands
(4) amplifying the circularized cDNA strands by rolling circle amplification, and
(5) synthesizing double-stranded or partially double-stranded, barcoded first complexes using the amplified cDNA strands as templates;
optionally wherein the method comprises fractioning the circularized cDNA strands to select circles of sizes within a predetermined range.

7. The method of any of the claims 1-6, wherein the method further comprises amplifying at least a portion of the barcoded first subfragments by
(1) releasing the barcoded first subfragments from first beads,
(2) denaturing barcoded first subfragments that have been released to form single-stranded barcoded first subfragments,
(3) circularizing the single-stranded barcoded first subfragments to produce circularized single DNA strands,
(4) performing rolling circle amplification on the circularized single DNA strands to produce amplified single-stranded barcoded first subfragments, and
(5) synthesizing double-stranded, barcoded first subfragments using the amplified single-stranded barcoded first subfragments as templates,
optionally wherein the method comprises fractioning the circularized single DNA strands to select circles of sizes within a predetermined range.

8. The method of any of the claims 1-7, wherein the method further comprises amplifying at least a portion of the barcoded first subfragments by
(1) extending the barcoded first subfragments using a primer binding to the primer binding sequence on the first capture oligonucleotide,
(2) releasing the extended barcoded first subfragments from (1) from the first beads,
(3) amplifying the barcoded first subfragments by about 10-120 fold using single primer amplification, thereby producing amplified double-stranded barcoded first subfragments, and
(4) ligating an adaptor oligonucleotides to the ends of the amplified double-stranded barcoded first subfragments.

9. The method of claim 1, wherein step (e) comprises ligating the at least some of the first subfragments in the first complexes with the first capture oligonucleotides.

10. The method of claim 1 wherein the double stranded or partially double stranded DNA molecules are bound to the plurality of first beads before step (d).

11. The method of claim 1, wherein the method further comprises amplifying at least a portion of the barcoded first subfragments to produce amplified barcoded first subfragments, wherein the amplified barcoded first subfragments are double-stranded or partially double-stranded;
(g) introducing staggered single-stranded breaks into some of the amplified barcoded first subfragments to generate second complexes, each comprising a plurality of second subfragments; and
(h) associating second capture oligonucleotide sequences with at least some of the second subfragments;
wherein the associating comprises combining the amplified barcoded first subfragments in (f) or the second complexes in (g) with a plurality of individual second beads, wherein each individual second bead comprises a plurality of second capture oligonucleotides immobilized thereon,
wherein each second capture oligonucleotide comprises a second capture oligonucleotide sequence,
wherein the second capture oligonucleotides immobilized on each individual second bead comprises the same second capture oligonucleotide sequence,
wherein a majority of different second beads have different second capture oligonucleotides immobilized thereon, and wherein each different second capture oligonucleotide sequence comprises a different second barcode,
thereby providing a library of barcoded second subfragments.

12. The method of claim 11, wherein
(i) amplifying at least a portion of the barcoded first subfragments is through linear amplification; or,
(ii) the average length of the first subfragments is at least 2x greater than the average length of second subfragments in size.

13. The method of claim 11, wherein step (h) is performed in a single mixture and wherein the number of second beads is greater than the number of the amplified barcoded first subfragments in the single mixture, wherein each second bead comprises multiple copies of the second capture oligo, immobilized thereon.

14. The method of claim 13 wherein each of at least some second subfragments is linked to a second insertion oligonucleotide, and wherein step (g) further comprises:
(1) ligating the second capture oligonucleotide to the second insertion oligonucleotide, or
(2) hybridizing the second capture oligonucleotide to the second insertion oligonucleotide and then extending the insertion oligonucleotide by a DNA polymerase to incorporate second barcodes.

15. The method of claim 8 and 11, wherein each of at least some second subfragments is linked to a second insertion oligonucleotide, and wherein step (e) further comprises:
(1) ligating the second capture oligonucleotide to the second insertion oligonucleotide, or
(2) hybridizing the second capture oligonucleotide to the second insertion oligonucleotide and then extending the insertion oligonucleotide by a DNA polymerase to incorporate second barcodes,
wherein the adaptor oligonucleotides have the same sequence as the second insertion oligonucleotide.

## Patentansprüche

1. Verfahren zum Vorbereiten einer Sequenzierungsbibliothek zum Sequenzieren einer Zielnukleinsäure, umfassend:
(a) Bereitstellen von (i) einzelsträngigen DNA-Kreisen der Zielnukleinsäure mit einer oder mehreren Kopien einer eindeutigen molekularen Kennung (UMI) und (ii) einer Vielzahl von ersten Kügelchen,
wobei jedes erste Kügelchen eine Vielzahl von ersten Einfangoligonukleotiden umfasst,
wobei jedes erste Einfangoligonukleotid einen ersten Barcode umfasst, der eine erste Barcode-Sequenz umfasst,
wobei alle ersten Einfangoligonukleotide, die auf demselben individuellen Kügelchen immobilisiert sind, dieselbe erste Barcode-Sequenz umfassen und eine Mehrheit der Kügelchen unterschiedliche erste Barcode-Sequenzen aufweist,
(b) Amplifizieren der einzelsträngigen DNA-Kreise aus (a) durch Rolling-Circle-Amplifikation, um eine Vielzahl von einzelsträngigen Konkatemeren zu produzieren, die zumindest fünf Kopien eines einzelsträngigen Fragments umfassen,
(c) Umwandeln der einzelsträngigen Konkatemere in doppelsträngige oder teilweise doppelsträngige DNA-Moleküle,
(d) Einführen von gestaffelten einzelsträngigen Brüchen in die DNA-Moleküle in (c), wodurch erste Komplexe erzeugt werden, die jeweils eine Vielzahl von ersten Subfragmenten von einem der DNA-Moleküle in (c) umfassen,
(e) Assoziieren von zumindest einigen der ersten Subfragmente in den ersten Komplexen mit ersten Einfangoligonukleotiden, die auf einer Vielzahl von individuellen ersten Kügelchen immobilisiert sind, wobei jedes individuelle erste Kügelchen eine Vielzahl der ersten Einfangoligonukleotide umfasst, wodurch barcodierte erste Subfragmente bereitgestellt werden.

2. Verfahren nach Anspruch 1, wobei zumindest einige erste Subfragmente mit ersten Insertionsoligonukleotiden verknüpft sind und wobei Schritt (e) ferner Folgendes umfasst:
(1) Ligieren der ersten Einfangoligonukleotide an die ersten Insertionsoligonukleotide, oder
(2) Hybridisieren der ersten Einfangoligonukleotide an die ersten Insertionsoligonukleotide und dann Erweitern der Insertionsoligonukleotide durch eine DNA-Polymerase, um erste Barcodes einzubauen.

3. Verfahren nach Anspruch 1, wobei Schritt (e) in einer einzelnen Mischung durchgeführt wird, wobei die Anzahl an ersten Kügelchen größer als die Anzahl an Zielnukleinsäurefragmenten in der einzelnen Mischung ist, und wobei jedes erste Kügelchen mehrere Kopien des ersten Einfangoligos, das darauf immobilisiert ist, umfasst.

4. Verfahren nach Anspruch 3, wobei erste Insertionsoligonukleotide durch Ligation oder durch Synthese zu zumindest einigen ersten Subfragmenten in Schritt (d) hinzugefügt werden und wobei Schritt (e) ferner Folgendes umfasst:
(1) Ligieren der ersten Einfangoligonukleotide an die ersten Insertionsoligonukleotide, oder
(2) Hybridisieren von ersten Einfangoligonukleotiden zu den ersten Insertionsoligonukleotiden und dann Erweitern der Insertionsoligonukleotide durch eine DNA-Polymerase, um erste Barcodes einzubauen.

5. Verfahren nach Anspruch 1, wobei:
(i) jedes erste Kügelchen mehrere Kopien des ersten Einfangoligonukleotids umfasst, wobei das erste Einfangoligonukleotid zu einem komplementären Oligonukleotid hybridisiert ist, um ein teilweise doppelsträngiges erstes Einfangoligonukleotid zu bilden,
wobei das Verfahren ferner Schritt (f) umfasst, umfassend
(1) Ligieren des ersten Einfangoligonukleotids an jedes von zumindest einigen der ersten Subfragmente durch 3'-Zweig-Ligation, oder
(2) Ligieren des komplementären Oligonukleotids an jedes von zumindest einigen der ersten Subfragmente durch 3'-Zweig-Ligation und Erweitern des komplementären Oligonukleotids, um die erste Barcode-Sequenz einzubauen; oder
(ii) die gestaffelten einzelsträngigen Brüche in die DNA-Moleküle durch Nickase eingeführt werden.

6. Verfahren nach einem der Ansprüche 1-5, wobei die ersten Einfangoligonukleotide ferner eine Promotorsequenz umfassen und wobei das Verfahren ferner Amplifizieren von zumindest einem Teil der barcodierten ersten Subfragmente durch Folgendes umfasst:
(1) Transkribieren der barcodierten ersten Komplexe, um RNA-Transkripte zu erzeugen,
(2) umgekehrtes Transkribieren der RNA-Transkripte unter Verwendung eines Primer-Annealings an die Promotorsequenz, um cDNA-Stränge der barcodierten ersten Komplexe zu erzeugen,
(3) Zirkularisieren der cDNA, um zirkularisierte cDNA-Stränge zu produzieren,
(4) Amplifizieren der zirkularisierten cDNA-Stränge durch Rolling-Circle-Amplifikation, und
(5) Synthetisieren von doppelsträngigen oder teilweise doppelsträngigen barcodierten ersten Komplexen unter Verwendung der amplifizierten cDNA-Stränge als Matrizen;
wobei optional das Verfahren Fraktionieren der zirkularisierten cDNA-Stränge umfasst, um Kreise mit Größen innerhalb eines vorbestimmten Bereichs auszuwählen.

7. Verfahren nach einem der Ansprüche 1-6, wobei das Verfahren ferner Amplifizieren von zumindest einem Teil der barcodierten ersten Subfragmente durch Folgendes umfasst:
(1) Freisetzen der barcodierten ersten Subfragmente von ersten Kügelchen,
(2) Denaturieren von barcodierten ersten Subfragmenten, die freigesetzt worden sind, um einzelsträngige barcodierte erste Subfragmente zu bilden,
(3) Zirkularisieren der einzelsträngigen barcodierten ersten Subfragmente, um zirkularisierte einzelne DNA-Stränge zu produzieren,
(4) Durchführen von Rolling-Circle-Amplifikation an den zirkularisierten einzelnen DNA-Strängen, um amplifizierte einzelsträngige barcodierte erste Subfragmente zu produzieren, und
(5) Synthetisieren von doppelsträngigen barcodierten ersten Subfragmenten unter Verwendung der amplifizierten einzelsträngigen barcodierten ersten Subfragmente als Matrizen,
wobei optional das Verfahren Fraktionieren der zirkularisierten einzelnen DNA-Stränge umfasst, um Kreise mit Größen innerhalb eines vorbestimmten Bereichs auszuwählen.

8. Verfahren nach einem der Ansprüche 1-7, wobei das Verfahren ferner Amplifizieren von zumindest einem Teil der barcodierten ersten Subfragmente durch Folgendes umfasst:
(1) Erweitern der barcodierten ersten Subfragmente unter Verwendung eines Primers, der an die Primer-Bindungssequenz an dem ersten Einfangoligonukleotid bindet,
(2) Freisetzen der erweiterten barcodierten ersten Subfragmente aus (1) von den ersten Kügelchen,
(3) Amplifizieren der barcodierten ersten Subfragmente um etwa das 10- bis 120-fache unter Verwendung von Einzelprimer-Amplifikation, wodurch amplifizierte doppelsträngige barcodierte erste Subfragmente produziert werden, und
(4) Ligieren von einem Adapter-Oligonukleotiden an die Enden der amplifizierten doppelsträngigen barcodierten ersten Subfragmente.

9. Verfahren nach Anspruch 1, wobei Schritt (e) Ligieren der zumindest einigen der ersten Subfragmente in den ersten Komplexen mit den ersten Einfangoligonukleotiden umfasst.

10. Verfahren nach Anspruch 1, wobei die doppelsträngigen oder teilweise doppelsträngigen DNA-Moleküle vor Schritt (d) an die Vielzahl von ersten Kügelchen gebunden werden.

11. Verfahren nach Anspruch 1, wobei das Verfahren ferner Amplifizieren von zumindest einem Teil der barcodierten ersten Subfragmente umfasst, um amplifizierte barcodierte erste Subfragmente zu produzieren, wobei die amplifizierten barcodierten ersten Subfragmente doppelsträngig oder teilweise doppelsträngig sind;
(g) Einführen von gestaffelten einzelsträngigen Brüchen in einige der amplifizierten barcodierten ersten Subfragmente, um zweite Komplexe zu erzeugen, die jeweils eine Vielzahl von zweiten Subfragmenten umfassen; und
(h) Assoziieren von zweiten Einfangoligonukleotidsequenzen mit zumindest einigen der zweiten Subfragmente;
wobei das Assoziieren Kombinieren der amplifizierten barcodierten ersten Subfragmente in (f) oder der zweiten Komplexe in (g) mit einer Vielzahl von individuellen zweiten Kügelchen umfasst, wobei jedes individuelle zweite Kügelchen eine Vielzahl von zweiten Einfangoligonukleotiden, die darauf immobilisiert ist, umfasst,
wobei jedes zweite Einfangoligonukleotid eine zweite Einfangoligonukleotidsequenz umfasst,
wobei die zweiten Einfangoligonukleotide, die auf jedem individuellen zweiten Kügelchen immobilisiert sind, dieselbe zweite Einfangoligonukleotidsequenz umfassen,
wobei eine Mehrzahl von unterschiedlichen zweiten Kügelchen unterschiedliche zweite Einfangoligonukleotide aufweist, die darauf immobilisiert sind, und wobei jede unterschiedliche zweite Einfangoligonukleotidsequenz einen unterschiedlichen zweiten Barcode umfasst,
wodurch eine Bibliothek aus barcodierten zweiten Subfragmenten bereitgestellt wird.

12. Verfahren nach Anspruch 11, wobei:
(i) Amplifizieren von zumindest einem Teil der barcodierten ersten Subfragmente durch lineare Amplifikation ist; oder
(ii) die durchschnittliche Länge der ersten Subfragmente zumindest 2x größer als die durchschnittliche Länge der zweiten Subfragmente in der Größe ist.

13. Verfahren nach Anspruch 11, wobei Schritt (h) in einer einzelnen Mischung durchgeführt wird und wobei die Anzahl an zweiten Kügelchen größer als die Anzahl der amplifizierten barcodierten ersten Subfragmente in der einzelnen Mischung ist, wobei jedes zweite Kügelchen mehrere Kopien des zweiten Einfangoligos, das darauf immobilisiert ist, umfasst.

14. Verfahren nach Anspruch 13, wobei jedes von zumindest einigen zweiten Subfragmenten mit einem zweiten Insertionsoligonukleotid verknüpft ist und wobei Schritt (g) ferner Folgendes umfasst:
(1) Ligieren des zweiten Einfangoligonukleotids an das zweite Insertionsoligonukleotid, oder
(2) Hybridisieren des zweiten Einfangoligonukleotids zu dem zweiten Insertionsoligonukleotid und dann Erweitern des Insertionsoligonukleotids durch eine DNA-Polymerase, um zweite Barcodes einzubauen.

15. Verfahren nach Anspruch 8 und 11, wobei jedes von zumindest einigen zweiten Subfragmenten mit einem zweiten Insertionsoligonukleotid verknüpft ist und wobei Schritt (e) ferner Folgendes umfasst:
(1) Ligieren des zweiten Einfangoligonukleotids an das zweite Insertionsoligonukleotid, oder
(2) Hybridisieren des zweiten Einfangoligonukleotids zu dem zweiten Insertionsoligonukleotid und dann Erweitern des Insertionsoligonukleotids durch eine DNA-Polymerase, um zweite Barcodes einzubauen,
wobei die Adapter-Oligonukleotide die gleiche Sequenz wie das zweite Insertionsoligonukleotid aufweisen.

## Revendications

1. Méthode permettant la préparation d'une banque de séquençage permettant de séquencer un acide nucléique cible comprenant :
(a) la fourniture (i) de cercles d'ADN simple brin de l'acide nucléique cible avec une ou plusieurs copies d'un identifiant moléculaire unique (UMI) et d'une pluralité de premières billes,
chaque première bille comprenant une pluralité de premiers oligonucléotides de capture,
chaque premier oligonucléotide de capture comprenant un premier code à barres comprenant une première séquence de codes à barres,
tous les premiers oligonucléotides de capture immobilisés sur la même bille individuelle comprenant la même séquence de code à barres, et une majorité de billes comportant des premières séquences de code à barres différentes,
(b) l'amplification des cercles d'ADN simple brin provenant de (a) par amplification par cercle roulant pour produire une pluralité de concatémères simple brin qui comprennent au moins cinq copies d'un fragment simple brin,
(c) la conversion des concatémères simple brin en molécules d'ADN double brin ou partiellement double brin,
(d) l'introduction de ruptures simple brin décalées dans les molécules d'ADN en (c), ce qui permet de générer des premiers complexes, chacun comprenant une pluralité de premiers sous-fragments provenant de l'une des molécules d'ADN en (c),
(e) l'association d'au moins certains des premiers sous-fragments dans les premiers complexes avec des premiers oligonucléotides de capture immobilisés sur une pluralité de premières billes individuelles, chaque première bille individuelle comprenant une pluralité de premiers oligonucléotides de capture, ce qui permet de fournir des premiers sous-fragments à code à barres.

2. Méthode selon la revendication 1, au moins certains premiers sous-fragments étant liés à des premiers oligonucléotides d'insertion, et ladite étape (e) comprenant en outre :
(1) la ligature des premiers oligonucléotides de capture aux premiers oligonucléotides d'insertion, ou
(2) l'hybridation des premiers oligonucléotides de capture aux premiers oligonucléotides d'insertion et ensuite l'extension des oligonucléotides d'insertion par une ADN polymérase pour incorporer des premiers codes à barres.

3. Méthode selon la revendication 1, ladite étape (e) étant réalisée dans un seul mélange, le nombre de premières billes étant supérieur au nombre de fragments d'acide nucléique cibles dans le seul mélange, et chaque première bille comprenant de multiples copies du premier oligonucléotide de capture, immobilisé sur celle-ci.

4. Méthode selon la revendication 3, lesdits premiers oligonucléotides d'insertion étant ajoutés par ligature ou par synthèse à au moins certains premiers sous-fragments à l'étape (d), et ladite étape (e) comprenant en outre :
(1) la ligature des premiers oligonucléotides de capture aux premiers oligonucléotides d'insertion, ou
(2) l'hybridation des premiers oligonucléotides de capture aux premiers oligonucléotides d'insertion et ensuite l'extension des oligonucléotides d'insertion par une ADN polymérase pour incorporer des premiers codes à barres.

5. Méthode selon la revendication 1 :
(i) chaque première bille comprenant de multiples copies du premier oligonucléotide de capture, ledit premier oligonucléotide de capture étant hybridé à un oligonucléotide complémentaire pour former un premier oligonucléotide de capture partiellement double brin,
ladite méthode comprenant en outre l'étape (f) comprenant
(1) la ligature du premier oligonucléotide de capture à chacun d'au moins certains des premiers sous-fragments par ligature de ramification 3', ou
(2) la ligature de l'oligonucléotide complémentaire à chacun d'au moins certains des premiers sous-fragments par ligature de ramification 3' et l'extension de l'oligonucléotide complémentaire pour incorporer la première séquence de codes à barres ; ou
(ii) les ruptures simple brin décalées étant introduites dans les molécules d'ADN par une nickase.

6. Méthode selon l'une quelconque des revendications 1 à 5, lesdits premiers oligonucléotides de capture comprenant en outre une séquence promotrice, et ladite méthode comprenant en outre l'amplification d'au moins une partie des premiers sous fragments à code à barres par
(1) transcription des premiers complexes à codes à barres pour générer des transcrits d'ARN,
(2) transcription inverse des transcrits d'ARN en utilisant un recuit d'amorces dans la séquence promotrice pour générer des brins d'ADNc des premiers complexes à codes à barres,
(3) circularisation de l'ADNc pour produire des brins d'ADNc circularisés,
(4) amplification des brins d'ADNc circularisés par amplification par cercle roulant, et
(5) synthèse de premiers complexes à codes à barres double brin ou partiellement double brin en utilisant les brins d'ADNc amplifiés comme matrices ;
éventuellement ladite méthode comprenant le fractionnement des brins d'ADNc circularisés pour sélectionner des cercles de tailles dans une plage prédéfinie.

7. Méthode selon l'une quelconque des revendications 1 à 6, ladite méthode comprenant en outre l'amplification d'au moins une partie des premiers sous-fragments à code à barres par
(1) libération des premiers sous-fragments à codes à barres des premières billes,
(2) dénaturation des premiers sous-fragments à code à barres qui ont été libérés pour former des premiers sous-fragments à code à barres simple brin,
(3) circularisation des premiers sous-fragments à code à barres simple brin pour produire des brins d'ADN uniques circularisés,
(4) réalisation d'une amplification par cercle roulant sur les brins d'ADN uniques circularisés pour produire des premiers sous-fragments à code à barres simple brin amplifiés, et
(5) synthèse de premiers sous-fragments à code à barres double brin en utilisant les premiers sous-fragments à code à barres simple brin amplifiés comme matrices,
éventuellement ladite méthode comprenant le fractionnement des brins d'ADN uniques circularisés pour sélectionner des cercles de tailles dans une plage prédéfinie.

8. Méthode selon l'une quelconque des revendications 1 à 7, ladite méthode comprenant en outre l'amplification d'au moins une partie des premiers sous-fragments à code à barres par
(1) extension des premiers sous-fragments à code à barres en utilisant une liaison d'amorce à la séquence de liaison d'amorce sur le premier oligonucléotide de capture,
(2) libération des premiers sous-fragments à code à barres étendus provenant de (1) des premières billes,
(3) amplification des premiers sous-fragments à code à barres d'environ 10 à 120 fois en utilisant une amplification par amorce unique, ce qui permet de produire des premiers sous-fragments à code à barres double brin amplifiés, et
(4) ligature d'un oligonucléotides adaptateurs aux extrémités des premiers sous-fragments à code à barres double brin amplifiés.

9. Méthode selon la revendication 1, ladite étape (e) comprenant la ligature desdits au moins certains des premiers sous-fragments dans les premiers complexes avec les premiers oligonucléotides de capture.

10. Méthode selon la revendication 1, lesdits molécules d'ADN double brin ou partiellement double brin étant liées à la pluralité de premières billes avant l'étape (d).

11. Méthode selon la revendication 1, ladite méthode comprenant en outre l'amplification d'au moins une partie des premiers sous-fragments à code à barres pour produire des premiers sous-fragments à code à barres amplifiés, lesdits premiers sous-fragments à code à barres amplifiés étant double brin ou partiellement double brin ;
(g) l'introduction de ruptures simple brin décalées dans certains des premiers sous-fragments à code à barres amplifiés pour générer des seconds complexes, chacun comprenant une pluralité de seconds sous-fragments ; et
(h) l'association de secondes séquences d'oligonucléotides de capture avec au moins certains des seconds sous-fragments ;
ladite association comprenant la combinaison des premiers sous-fragments à code à barres amplifiés en (f) ou des seconds complexes en (g) avec une pluralité de secondes billes individuelles, chaque seconde bille individuelle comprenant une pluralité de seconds oligonucléotides de capture immobilisés sur celle-ci,
chaque second oligonucléotide de capture comprenant une seconde séquence d'oligonucléotide de capture,
chaque second oligonucléotide de capture immobilisé sur chaque seconde bille individuelle comprenant la même seconde séquence d'oligonucléotide de capture,
une majorité de secondes billes différentes comportant des seconds oligonucléotides de capture différents immobilisés sur celles-ci, et chaque seconde séquence d'oligonucléotide de capture différente comprenant un second code à barres différent,
ce qui permet de fournir une banque de seconds sous-fragments à code à barres.

12. Méthode selon la revendication 11
(i) ladite amplification d'au moins une partie des premiers sous-fragments à code à barres se faisant par une amplification linéaire ; ou,
(ii) la longueur moyenne des premiers sous-fragments étant au moins 2x plus grande que la longueur moyenne des seconds sous-fragments en taille.

13. Méthode selon la revendication 11, ladite étape (h) étant réalisée dans un seul mélange et le nombre de secondes billes étant supérieur au nombre des premiers sous-fragments à code à barres amplifiés dans le seul mélange, chaque seconde bille comprenant de multiples copies du second oligonucléotide de capture, immobilisé sur celles-ci.

14. Méthode selon la revendication 13, chacun d'au moins certains des seconds sous-fragments étant lié à un second oligonucléotide d'insertion, et ladite étape (g) comprenant en outre :
(1) la ligature du second oligonucléotide de capture au second oligonucléotide d'insertion, ou
(2) l'hybridation du second oligonucléotide de capture au second oligonucléotide d'insertion et ensuite l'extension de l'oligonucléotide d'insertion par une ADN polymérase pour incorporer des seconds codes à barres.

15. Méthode selon les revendications 8 et 11, chacun d'au moins certains seconds fragments étant lié à un second oligonucléotide d'insertion, et ladite étape (e) comprenant en outre :
(1) la ligature du second oligonucléotide de capture au second oligonucléotide d'insertion, ou
(2) l'hybridation du second oligonucléotide de capture au second oligonucléotide d'insertion et ensuite l'extension de l'oligonucléotide d'insertion par une ADN polymérase pour incorporer des seconds codes à barres,
lesdits oligonucléotides adaptateurs comportant la même séquence que le second oligonucléotide d'insertion.
